# EUROPEAN PATENT APPLICATION

(11) **EP 4 755 921 A1**
(43) Date of publication of application: **10.06.2026**
(21) Application number: 24851144.6
(22) Date of filing: 09.08.2024
(51) Int. Cl.: C07K 16/46, C12N 15/13, A61K 39/395, A61P 35/00

(54) **GPRC5D ANTIBODY-CONTAINING CHIMERIC ANTIGEN RECEPTOR AND USE THEREOF**

(30) Priority: 10.08.2023 CN 202311010359
(71) Applicant: Shenzhen Cell Inspire Biotechnology Co., Ltd., Shenzhen, Guangdong 518107 (CN)
(72) Inventor: YANG, Jiayin, Shenzhen, Guangdong 518107 (CN); FU, Jian, Shenzhen, Guangdong 518107 (CN); JIANG, Lixiang, Shenzhen, Guangdong 518107 (CN); ZHU, Zhibin, Shenzhen, Guangdong 518107 (CN); YAN, Yubo, Shenzhen, Guangdong 518107 (CN); WEI, Mingjun, Shenzhen, Guangdong 518107 (CN)
(74) Representative: Potter Clarkson
(86) International application number: PCT/CN2024/111243
(87) International publication number: WO 2025/031500

(57) **Abstract**

Provided are a chimeric antigen receptor, and a GPRC5D antibody-containing chimeric antigen receptor and use thereof. The chimeric antigen receptor includes: an extracellular domain, capable of binding to a tumor antigen; a transmembrane domain, linked to the extracellular domain, the transmembrane domain being from a type I transmembrane protein or is an artificially synthesized transmembrane protein with a hydrophobic helix; and an intracellular domain, linked to the transmembrane domain. The tumor antigen is a tumor-specific antigen or a tumor-associated antigen. The amino acid sequence of the transmembrane domain is optimized, thereby providing the chimeric antigen receptor containing the GPRC5D antibody and an engineered immune cell. The engineered immune cell obtained based on the GPRC5D antibody exhibits a specific killing activity against multiple myeloma.

## Description

### PRIORITY INFORMATION

The present application claims priority to Chinese patent application No. 202311010359.2, titled "GPRC5D ANTIBODY-CONTAINING CHIMERIC ANTIGEN RECEPTOR AND USE THEREOF" and filed on August 10, 2023, the entire contents of which are incorporated herein by reference.

### FIELD

The present disclosure relates to the field of biotechnology, and more particularly, to a chimeric antigen receptor, a chimeric antigen receptor containing a GPRC5D antibody, a polynucleotide, an expression vector, a cell, a composition, pharmaceutical use, and a kit for detecting GPRC5D.

### BACKGROUND

Multiple myeloma (MM) is a malignant disease characterized by the abnormal proliferation of clonal plasma cells. In many countries, it is the second most common hematological malignancy, predominantly affecting the elderly, and remains currently incurable. In China, the incidence of multiple myeloma has exceeded that of acute leukemia, with a rate of approximately 1 per 100,000 population. With the continuous intensification of population aging in China, the number of patients is expected to rise further. In recent years, therapeutic strategies have been significantly improved. Representative examples include monoclonal antibody therapy represented by daratumumab (an anti-CD38 antibody), bispecific antibody therapy represented by Tecvayli (BCMAxCD3), and immune cell therapy represented by chimeric antigen receptor T (CAR-T) cells.

G protein-coupled receptor family C group 5 member D (GPRC5D), an orphan receptor, is a seven-pass transmembrane protein that is specifically highly expressed in multiple myeloma cells, while expressed at low levels or not expressed at all in normal tissues. Therefore, this target is expected to become a novel therapeutic target for MM.

Chimeric antigen receptors (CARs) can activate immune cells to specifically kill tumors. Previous studies have found that the transmembrane domain of CAR is crucial for immune cell activation (https://doi.org/10.1016/j.stem.2018.06.002), and the transmembrane domain from the NKG2D transmembrane region can enhance the tumor-killing activity of CAR-NK cells. To further improve the tumor-killing activity, it is urgently needed to optimize the transmembrane domain and the structure of chimeric antigen receptors.

### SUMMARY

The present disclosure aims to solve at least one of the technical problems in the related art to a certain extent. To this end, an objective of the present disclosure is to provide a chimeric antigen receptor, a chimeric antigen receptor containing a GPRC5D antibody, and an engineered immune cell. The engineered immune cell obtained based on the GPRC5D antibody exhibits specific killing activity against multiple myeloma.

To this end, in a first aspect of the present disclosure, the present disclosure provides a chimeric antigen receptor. According to an embodiment of the present disclosure, the chimeric antigen receptor includes: an extracellular domain, capable of binding to a tumor antigen, an inflammatory factor, or a pathogen antigen; a transmembrane domain, linked to the extracellular domain, the transmembrane domain being from a type I transmembrane protein or an artificially synthesized transmembrane protein with a hydrophobic helix; and an intracellular domain, linked to the transmembrane domain. The amino acid sequence of the transmembrane domain is SNLFVASWIAVMX₈IFX₉IGX₁₀AX₁₁AX₁₂X₁₃X₁₄X₁₅X₁₆X₁₇X₁₈X₁₉PS. X₈ is selected from I or V, X₉ is selected from R or H, X₁₀ is selected from M or L, X₁₁ is selected from V or I, X₁₂ is selected from I or V, X₁₃ is selected from S or F, X₁₄ is selected from C, R, or H, X₁₅ is selected from C, T, or V, X₁₆ is selected from V, L, or absent, X₁₇ is selected from F or H, X₁₈ is selected from F or Y, X₁₉ is selected from L or absent, and X₁₆ and X₁₉ are not present simultaneously and X₁₆ and X₁₉ are not absent simultaneously. The tumor antigen is a tumor-specific antigen or a tumor-associated antigen.

The present disclosure further optimizes the amino acid sequence of the transmembrane domain. When the transmembrane domain of the chimeric antigen receptor has the amino acid sequence as set forth in the above-described general formula, the engineered immune cell containing the chimeric antigen receptor exhibits an enhanced ability to kill a target cell (a cell which the extracellular domain targets and binds to).

According to an embodiment of the present disclosure, the transmembrane domain has the amino acid sequence as set forth in any one of SEQ ID NO: 19 to SEQ ID NO: 23 and SEQ ID NO: 27 to SEQ ID NO: 29.
SNLFVASWIAVMIIFRIGMAVAIFCCFFLPS (SEQ ID NO: 19).
SNLFVASWIAVMVIFRIGMAVAIFRCLFFPS (SEQ ID NO: 20).
SNLFVASWIAVMIIFRIGMAVAIFCCLFFPS (SEQ ID NO: 21).
SNLFVASWIAVMIIFHIGLAIAISHVLFFPS (SEQ ID NO: 22).
SNLFVASWIAVMIIFHIGMAIAISRCLFFPS (SEQ ID NO: 23).
SNLFVASWIAVMIIFHIGMAIAISCTVFFPS (SEQ ID NO: 27).
SNLFVASWIAVMIIFRIGMAVAVSRCLHFPS (SEQ ID NO: 28).
SNLFVASWIAVMIIFHIGLAIAISRCLFYPS (SEQ ID NO: 29).

According to an embodiment of the present disclosure, the extracellular domain includes an antibody or antigen-binding fragment thereof capable of specifically recognizing a tumor antigen, an inflammatory factor, or a pathogen antigen.

In a second aspect of the present disclosure, the present disclosure provides a chimeric antigen receptor. According to an embodiment of the present disclosure, the chimeric antigen receptor includes: an extracellular domain, capable of binding to a tumor antigen; a transmembrane domain, linked to the extracellular domain, the transmembrane domain being from a type I transmembrane protein or an artificially synthesized transmembrane protein with a hydrophobic helix; and an intracellular domain, linked to the transmembrane domain. The amino acid sequence of the transmembrane domain is SNLFVASWIAVMX₁IFX₂IGMAX₃AIX₄X₅CX₆FFX₇PS. X₁ is selected from I or V, X₂ is selected from R or H, X₃ is selected from V or I, X₄ is selected from S or F, X₅ is selected from C or R, X₆ is selected from L or absent, and X₇ is selected from L or absent. The tumor antigen is a tumor-specific antigen or a tumor-associated antigen.

The present disclosure further optimizes the amino acid sequence of the transmembrane domain. When the transmembrane domain of the chimeric antigen receptor has the amino acid sequence as set forth in the above-described general formula, the engineered immune cell containing the chimeric antigen receptor exhibits an enhanced ability to kill a target cell (a cell which the extracellular domain targets and binds to).

According to an embodiment of the present disclosure, the transmembrane domain has the amino acid sequence as set forth in any one of SEQ ID NO: 19 to SEQ ID NO: 21 and SEQ ID NO: 23.

According to an embodiment of the present disclosure, the extracellular domain includes an antibody or antigen-binding fragment thereof capable of specifically recognizing a tumor antigen.

In a third aspect of the present disclosure, the present disclosure provides a chimeric antigen receptor. According to an embodiment of the present disclosure, the chimeric antigen receptor includes: an extracellular domain, capable of binding to a tumor antigen, an inflammatory factor, or a pathogen antigen; a transmembrane domain, linked to the extracellular domain, the transmembrane domain being from a type I transmembrane protein or an artificially synthesized transmembrane protein with a hydrophobic helix; and an intracellular domain, linked to the transmembrane domain. The amino acid sequence of the transmembrane domain is SNLFVASWIAVMIIFX₂₀IGX₂₁AX₂₂AX₂₃SX₂₄X₂₅X₂₆X₂₇X₂₈PS. X₂₀ is selected from R or H, X₂₁ is selected from M or L, X₂₂ is selected from V or I, X₂₃ is selected from V or I, X₂₄ is selected from C, R, or H, X₂₅ is selected from C, T, or V, X₂₆ is selected from L or V, X₂₇ is selected from F or H, and X₂₈ is selected from F or Y. The tumor antigen is a tumor-specific antigen or a tumor-associated antigen.

The present disclosure further optimizes the amino acid sequence of the transmembrane domain. When the transmembrane domain of the chimeric antigen receptor has the amino acid sequence as set forth in the above-described general formula, the engineered immune cell containing the chimeric antigen receptor exhibits an enhanced ability to kill a target cell (a cell which the extracellular domain targets and binds to).

According to an embodiment of the present disclosure, the transmembrane domain has the amino acid sequence as set forth in any one of SEQ ID NO: 22 and SEQ ID NO: 27 to SEQ ID NO: 29.

According to an embodiment of the present disclosure, the extracellular domain includes an antibody or antigen-binding fragment thereof capable of specifically recognizing a tumor antigen, an inflammatory factor, or a pathogen antigen.

According to an embodiment of the present disclosure, the above-described chimeric antigen receptor in the first aspect, the second aspect, or the third aspect may further include at least one of the following additional technical features:

According to an embodiment of the present disclosure, the extracellular domain includes an antibody or antigen-binding fragment thereof capable of specifically recognizing at least one selected from the group consisting of: CD19, BCMA, GPRC5D, CD123, CD138, CD20, CD22, CD30, CD33, CD38, CD4, CD7, CLL-1, CD10, CD34, CS1, CD16, CD5, IL-1-RAP, ITGB7, k-IgG, TACI, TRBC1, MUC1, NKG2DL, PD-L1, CD133, CD117, LeY, CD70, ROR1, CD147, CD171, CD267, AXL, B7-H3, CD44v6, CEA, CAIX, CLDN18.2, CLDN6, DLL3, DR5, EGFR, EpCAM, ErbB, FAP, FBP, FRα, GD2, gp100, HER2, IL-13Rα2, LFA1, LMP1, MAGE-A3, MAGE-A1, MAGE-A4, MART1, MET, MG7, MMP2, MSLN, MUC1, MUC16, MUC16ecto, NECTIN4, NKG2D, PMEL, PSCA, PSMA, ROR1, ROR2, TM4SF1, TnMUC1, VEGFR2, CLD18, and GPC3.

According to an embodiment of the present disclosure, the extracellular domain further includes an antibody or antigen-binding fragment thereof capable of specifically recognizing a pathogen antigen. The pathogen includes EBV.

According to an embodiment of the present disclosure, the extracellular domain further includes an antibody or antigen-binding fragment thereof capable of specifically recognizing an inflammatory factor. The inflammatory factor includes at least one selected from the group consisting of: IL-1, TNF-α, IFN-γ, IL-12, IL-18, GMCSF, IL-6, IL-8, IL-17, CXCL1, CXCL2, CXCL9, CXCL10, CXCL11, CXCL16, and CCL2-2.

According to an embodiment of the present disclosure, the extracellular domain includes an antibody or antigen-binding fragment thereof capable of specifically recognizing GPRC5D.

According to an embodiment of the present disclosure, the antibody or the antigen-binding fragment capable of specifically recognizing GPRC5D includes at least one CDR sequence selected from the following or an amino acid sequence having at least 90% identity thereto:
heavy chain variable region CDR sequences: SEQ ID NO: 1 to SEQ ID NO:3; and
light chain variable region CDR sequences: SEQ ID NO: 4, the amino acid sequence SAS, and SEQ ID NO: 5.
GYSITSDYA (SEQ ID NO: 1).
ISYSGSA (SEQ ID NO: 2).
ARGGIAGRGRWGAMDY (SEQ ID NO: 3).
QNVGTN (SEQ ID NO: 4).
CDR 2 sequence of the light chain variable region: SAS.
QQYKSYPLT (SEQ ID NO: 5).

The present disclosure provides a chimeric antigen receptor with an optimized transmembrane domain and a novel GPRC5D antibody that specifically binds to GPRC5D in the extracellular domain. The engineered immune cell designed based on the GPRC5D antibody of the present disclosure can specifically target multiple myeloma cells, kill target cells, and prevent relapse.

According to an embodiment of the present disclosure, the antibody or the antigen-binding fragment capable of specifically recognizing GPRC5D includes:
heavy chain variable region CDR1, CDR2, and CDR3 sequences as set forth in SEQ ID NO: 1, SEQ ID NO: 2, and SEQ ID NO: 3, respectively, or amino acid sequences having at least 90% identity thereto.

According to an embodiment of the present disclosure, the antibody or the antigen-binding fragment capable of specifically recognizing GPRC5D includes:
light chain variable region CDR1, CDR2, and CDR3 sequences as set forth in SEQ ID NO: 4, the amino acid sequence SAS, and SEQ ID NO: 5, respectively, or amino acid sequences having at least 90% identity thereto.

According to an embodiment of the present disclosure, the antibody or the antigen-binding fragment capable of specifically recognizing GPRC5D includes: heavy chain variable region CDR1, CDR2, and CDR3 sequences as set forth in SEQ ID NO: 1, SEQ ID NO: 2, and SEQ ID NO: 3, respectively, or amino acid sequences having at least 90% identity thereto, and light chain variable region CDR1, CDR2, and CDR3 sequences as set forth in SEQ ID NO: 4, the amino acid sequence SAS, and SEQ ID NO: 5, respectively, or amino acid sequences having at least 90% identity thereto.

The GPRC5D antibody or antigen-binding fragment thereof according to an embodiment of the present disclosure has the heavy chain variable region CDR1, CDR2, CDR3 sequences as set forth in SEQ ID NO: 1, SEQ ID NO: 2 and SEQ ID NO: 3, respectively, and the light chain variable region CDR1, CDR2, and CDR3 sequences as set forth in SEQ ID NO: 4, the amino acid sequence SAS, and SEQ ID NO: 5, respectively. The antibody is capable of specifically binding to GPRC5D on surfaces of multiple myeloma cells. The engineered immune cell designed based on the antibody can specifically target multiple myeloma cells, kill target cells, and prevent relapse.

According to an embodiment of the present disclosure, the antibody or the antigen-binding fragment capable of specifically recognizing GPRC5D includes a heavy chain variable region having the amino acid sequence as set forth in SEQ ID NO: 6.

According to an embodiment of the present disclosure, the antibody or the antigen-binding fragment capable of specifically recognizing GPRC5D includes a light chain variable region having the amino acid sequence as set forth in SEQ ID NO: 7.

According to an embodiment of the present disclosure, the antibody is a single-chain antibody having the amino acid sequence as set forth in SEQ ID NO: 8.

QVQLQESGPGLVKPSETLSLTCTVSGYSITSDYAWNWIRQPPGKGLEWMG
YISYSGSATYSPSLKSRITISRDTSKNQFSLKLSSVTAADTAVYYCARGGIAGRGRWGA
MDYWGQGTTVTVSS (SEQ ID NO: 6).

DIQMTQSPSSLSASVGDRVTITCKASQNVGTNVAWYQQKPGQSPKALIYSA
SYRDSGVPDRFSGSGSGTDFTLTISSLQPEDFATYYCQQYKSYPLTFGQGTRLEIK (SEQ
ID NO: 7).

DIQMTQSPSSLSASVGDRVTITCKASQNVGTNVAWYQQKPGQSPKALIYSA
SYRDSGVPDRFSGSGSGTDFTLTISSLQPEDFATYYCQQYKSYPLTFGQGTRLEIKGGG

GSGGGGSGGGGSQVQLQESGPGLVKPSETLSLTCTVSGYSITSDYAWNWIRQPPGKGL
EWMGYISYSGSATYSPSLKSRITISRDTSKNQFSLKLSSVTAADTAVYYCARGGIAGRG
RWGAMDYWGQGTTVTVSS (SEQ ID NO: 8).

According to an embodiment of the present disclosure, the extracellular domain includes a bispecific binding molecule. The bispecific binding molecule includes:
a first binding region, including the antibody or antigen-binding fragment thereof capable of specifically recognizing GPRC5D; and
a second binding region, having an activity of binding to an antigen specifically expressed on lymphocytes.

According to an embodiment of the present disclosure, the antigen specifically expressed on lymphocytes includes at least one selected from the group consisting of BCMA, CD38, CD138, CD3E, and FCGR3A.

According to an embodiment of the present disclosure, the second binding region includes a single-chain antibody or a single-domain antibody.

According to an embodiment of the present disclosure, the second binding region has BCMA-binding activity.

According to an embodiment of the present disclosure, the second binding region includes a single-chain antibody or a single-domain antibody capable of specifically recognizing BCMA.

According to an embodiment of the present disclosure, the second binding region has the amino acid sequence as set forth in SEQ ID NO: 9.

QVKLEESGGGLVQAGRSLRLSCAASEHTFSSHVMGWFRQAPGKERESVAV
IGWRDISTSYADSVKGRFTISRDNAKKTLYLQMNSLKPEDTAVYYCAARRIDAADFDS
WGQGTQVTVSS (SEQ ID NO: 9).

According to an embodiment of the present disclosure, the first binding region is linked to the second binding region via a linking peptide.

According to an embodiment of the present disclosure, the bispecific binding molecule has the amino acid sequence as set forth in SEQ ID NO: 10.

DIQMTQSPSSLSASVGDRVTITCKASQNVGTNVAWYQQKPGQSPKALIYSA
SYRDSGVPDRFSGSGSGTDFTLTISSLQPEDFATYYCQQYKSYPLTFGQGTRLEIKGGG

GSGGGGSGGGGSQVQLQESGPGLVKPSETLSLTCTVSGYSITSDYAWNWIRQPPGKGL
EWMGYISYSGSATYSPSLKSRITISRDTSKNQFSLKLSSVTAADTAVYYCARGGIAGRG
RWGAMDYWGQGTTVTVSSGGGGSGGGGSGGGGSGGGGSGGGGSQVKLEESGGGL
VQAGRSLRLSCAASEHTFSSHVMGWFRQAPGKERESVAVIGWRDISTSYADSVKGRF
TISRDNAKKTLYLQMNSLKPEDTAVYYCAARRIDAADFDSWGQGTQVTVSS (SEQ ID
NO: 10).

Existing bispecific antibodies targeting BCMA or CAR-T therapy have achieved some efficacy in the treatment of MM. However, due to BCMA antigen escape, relapse is inevitable in MM patients. The inventors have discovered that by combining the GPRC5D antibody of the present disclosure with a BCMA antibody to form a bispecific antibody, the engineered immune cell designed based on this bispecific antibody can specifically target multiple myeloma cells, kill target cells, and prevent relapse, solving the problem of BCMA antigen escape.

According to an embodiment of the present disclosure, the extracellular domain is linked to the transmembrane domain via a hinge domain.

According to an embodiment of the present disclosure, the hinge domain is from a hinge region of at least one protein selected from the group consisting of:

CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT.

According to an embodiment of the present disclosure, the hinge domain is from a hinge region of CD8.

According to an embodiment of the present disclosure, the hinge domain has the amino acid sequence as set forth in SEQ ID NO: 11.
TTTPAPRPPTPAPTIASQPLSLRPEACRPAAGGAVHTRGLDFACD (SEQ ID NO: 11).

According to an embodiment of the present disclosure, the intracellular domain includes a co-stimulatory signal domain and an intracellular signaling domain. The co-stimulatory signal domain is linked to the intracellular signaling domain via a linking peptide.

According to an embodiment of the present disclosure, the co-stimulatory signal domain includes an intracellular co-stimulatory signal domain from at least one protein selected from the group consisting of:
CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88.

According to an embodiment of the present disclosure, the co-stimulatory signal domain is from an intracellular co-stimulatory signal domain of 2B4.

According to an embodiment of the present disclosure, the co-stimulatory signal domain has the amino acid sequence as set forth in SEQ ID NO: 12.

WRRKRKEKQSETSPKEFLTIYEDVKDLKTRRNHEQEQTFPGGGSTIYSMIQ
SQSSAPTSQEPAYTLYSLIQPSRKSGSRKRNHSPSFNSTIYEVIGKSQPKAQNPARLSRK
ELENFDVYS (SEQ ID NO: 12).

According to an embodiment of the present disclosure, the intracellular signaling domain includes at least one ITAM motif.

According to an embodiment of the present disclosure, the intracellular signaling domain includes an intracellular signaling domain from at least one protein selected from the group consisting of:
CD3ζ, CD3δ, CD3γ, CD3ε, CD79a, CD79b, FcεRIγ, FcεRIβ, FcγRIIa, DAP10, and DAP-12.

According to an embodiment of the present disclosure, the intracellular signaling domain is an intracellular signaling domain from CD3ζ.

According to an embodiment of the present disclosure, the intracellular signaling domain has the amino acid sequence as set forth in SEQ ID NO: 13.

RVKFSRSADAPAYKQGQNQLYNELNLGRREEYDVLDKRRGRDPEMGGKP
RRKNPQEGLYNELQKDKMAEAYSEIGMKGERRRGKGHDGLYQGLSTATKDTYDALH
MQALPPR (SEQ ID NO: 13).

According to an embodiment of the present disclosure, the chimeric antigen receptor further includes a signal peptide linked to the N-terminus of the extracellular domain of the chimeric antigen receptor.

According to an embodiment of the present disclosure, the signal peptide is from CD8 protein.

According to an embodiment of the present disclosure, the signal peptide has the amino acid sequence as set forth in SEQ ID NO: 14.
MALPVTALLLPLALLLHAARP (SEQ ID NO: 14).

According to an embodiment of the present disclosure, the chimeric antigen receptor has the amino acid sequence as set forth in SEQ ID NO: 15 or SEQ ID NO:16, with the transmembrane domain sequence underlined. For the transmembrane domain of the chimeric antigen receptor in the present disclosure, the transmembrane domains as set forth in SEQ ID NO: 18 to SEQ ID NO: 23 may be used according to different experimental requirements. SEQ ID NO: 18 is the non-mutated amino acid sequence of the transmembrane domain.

MALPVTALLLPLALLLHAARPDIQMTQSPSSLSASVGDRVTITCKASQNVG
TNVAWYQQKPGQSPKALIYSASYRDSGVPDRFSGSGSGTDFTLTISSLQPEDFATYYCQ
QYKSYPLTFGQGTRLEIKGGGGSGGGGSGGGGSQVQLQESGPGLVKPSETLSLTCTVS
GYSITSDYAWNWIRQPPGKGLEWMGYISYSGSATYSPSLKSRITISRDTSKNQFSLKLS
SVTAADTAVYYCARGGIAGRGRWGAMDYWGQGTTVTVSSTTTPAPRPPTPAPTIASQ
PLSLRPEACRPAAGGAVHTRGLDFACDSNLFVASWIAVMIIFRIGMAVAIFCCFFLPSWR
RKRKEKQSETSPKEFLTIYEDVKDLKTRRNHEQEQTFPGGGSTIYSMIQSQSSAPTSQE
PAYTLYSLIQPSRKSGSRKRNHSPSFNSTIYEVIGKSQPKAQNPARLSRKELENFDVYSR
VKFSRSADAPAYKQGQNQLYNELNLGRREEYDVLDKRRGRDPEMGGKPRRKNPQEG
LYNELQKDKMAEAYSEIGMKGERRRGKGHDGLYQGLSTATKDTYDALHMQALPPR
(SEQ ID NO: 15).

MALPVTALLLPLALLLHAARPDIQMTQSPSSLSASVGDRVTITCKASQNVG
TNVAWYQQKPGQSPKALIYSASYRDSGVPDRFSGSGSGTDFTLTISSLQPEDFATYYCQ
QYKSYPLTFGQGTRLEIKGGGGSGGGGSGGGGSQVQLQESGPGLVKPSETLSLTCTVS
GYSITSDYAWNWIRQPPGKGLEWMGYISYSGSATYSPSLKSRITISRDTSKNQFSLKLS
SVTAADTAVYYCARGGIAGRGRWGAMDYWGQGTTVTVSSGGGGSGGGGSGGGGS
GGGGSGGGGSQVKLEESGGGLVQAGRSLRLSCAASEHTFSSHVMGWFRQAPGKERE

SVAVIGWRDISTSYADSVKGRFTISRDNAKKTLYLQMNSLKPEDTAVYYCAARRIDAA
DFDSWGQGTQVTVSSTTTPAPRPPTPAPTIASQPLSLRPEACRPAAGGAVHTRGLDFAC
DSNLFVASWIAVMIIFRIGMAVAIFCCFFLPSWRRKRKEKQSETSPKEFLTIYEDVKDLK
TRRNHEQEQTFPGGGSTIYSMIQSQSSAPTSQEPAYTLYSLIQPSRKSGSRKRNHSPSFN
STIYEVIGKSQPKAQNPARLSRKELENFDVYSRVKFSRSADAPAYKQGQNQLYNELNL
GRREEYDVLDKRRGRDPEMGGKPRRKNPQEGLYNELQKDKMAEAYSEIGMKGERR
RGKGHDGLYQGLSTATKDTYDALHMQALPPR (SEQ ID NO: 16).

In a fourth aspect of the present disclosure, the present disclosure provides an isolated polynucleotide. According to an embodiment of the present disclosure, the isolated polynucleotide encodes the chimeric antigen receptor in the first aspect, the second aspect, or the third aspect.

In a fifth aspect of the present disclosure, the present disclosure provides an expression vector. According to an embodiment of the present disclosure, the expression vector carries the polynucleotide in the fourth aspect.

In a sixth aspect of the present disclosure, the present disclosure provides a cell. According to an embodiment of the present disclosure, the cell carries the polynucleotide in the fourth aspect or the expression vector in the fifth aspect, or is capable of expressing the chimeric antigen receptor in the first aspect, the second aspect, or the third aspect.

According to an embodiment of the present disclosure, the cell includes an immune cell.

According to an embodiment of the present disclosure, the immune cell is isolated from peripheral blood, umbilical cord blood, or tissues or organs, or is differentiated from a stem cell or an immune progenitor cell.

According to an embodiment of the present disclosure, the stem cell includes an induced pluripotent stem cell, a hematopoietic stem cell, or an embryonic stem cell within 14 days after fertilization and not subjected to in vivo development.

According to an embodiment of the present disclosure, the immune cell includes at least one selected from the group consisting of an NK cell, a T cell, and a macrophage.

In a seventh aspect, the present disclosure provides a composition. According to an embodiment of the present disclosure, the composition includes at least one of the chimeric antigen receptor in the first aspect, the second aspect, or the third aspect, the polynucleotide in the fourth aspect, the expression vector in the fifth aspect, or the cell in the sixth aspect.

In an eighth aspect, the present disclosure provides use of the composition in the seventh aspect in the manufacture of a medicament for treating a disease.

According to an embodiment of the present disclosure, the disease is a hematologic malignancy or solid tumor, an autoimmune disease, or a viral infectious disease.

According to an embodiment of the present disclosure, the hematologic malignancy or solid tumor is a GPRC5D-associated disease.

According to an embodiment of the present disclosure, the GPRC5D-associated disease includes multiple myeloma.

In a ninth aspect, the present disclosure provides a kit for detecting GPRC5D. According to an embodiment of the present disclosure, the kit includes the chimeric antigen receptor in the first aspect, the second aspect, or the third aspect.

In a tenth aspect, the present disclosure provides use of the antibody or antigen-binding fragment thereof in the first aspect, the second aspect, or the third aspect, the polynucleotide in the fourth aspect, or the expression vector in the fifth aspect in the manufacture of a kit. According to an embodiment of the present disclosure, the kit is for use in detecting GPRC5D or diagnosing a GPRC5D-associated disease.

According to an embodiment of the present disclosure, the GPRC5D-associated disease includes multiple myeloma.

In an eleventh aspect, the present disclosure provides use of the chimeric antigen receptor in the first aspect, the second aspect, or the third aspect, the polynucleotide in the fourth aspect, the expression vector in the fifth aspect, the cell in the sixth aspect, and/or the composition in the seventh aspect in the treatment of a hematologic malignancy or solid tumor, an autoimmune disease, or a viral infectious disease in a subject.

According to an embodiment of the present disclosure, the hematologic malignancy or solid tumor is a GPRC5D-associated disease.

According to an embodiment of the present disclosure, the GPRC5D-associated disease includes multiple myeloma.

In the twelfth aspect of the present disclosure, the present disclosure provides a method for preventing and/or treating a hematologic malignancy or solid tumor, an autoimmune disease, or a viral infectious disease in a subject. According to an embodiment of the present disclosure, the method includes administering to the subject at least one of the chimeric antigen receptor in the first aspect, the second aspect, or the third aspect, the polynucleotide in the fourth aspect, the expression vector in the fifth aspect, the cell in the sixth aspect, or the composition in the seventh aspect.

According to an embodiment of the present disclosure, the hematologic malignancy or solid tumor is a GPRC5D-associated disease.

According to an embodiment of the present disclosure, the GPRC5D-associated disease includes multiple myeloma.

In a thirteenth aspect of the present disclosure, the present disclosure provides use of the chimeric antigen receptor in the first aspect, the second aspect, or the third aspect, the polynucleotide in the fourth aspect, or the expression vector in the fifth aspect in the detection of GPRC5D or in the diagnosis of whether a subject is suffering from a GPRC5D-associated disease.

According to an embodiment of the present disclosure, the GPRC5D-associated disease includes multiple myeloma.

In a fourteenth aspect of the present disclosure, the present disclosure provides a method for detecting GPRC5D. According to an embodiment of the present disclosure, the method includes detecting GPRC5D in a sample to be tested using at least one of the chimeric antigen receptor in the first aspect, the second aspect, or the third aspect, the polynucleotide in the fourth aspect, or the expression vector in the fifth aspect.

The method for detecting GPRC5D according to an embodiment of the present disclosure may be used for scientific research. The sample includes, but is not limited to, tissues, cells, blood, serum, plasma, urine, feces, saliva, or sweat.

According to a specific embodiment of the present disclosure, the present disclosure provides a method for diagnosing whether a subject is suffering from a GPRC5D-associated disease. The method includes detecting GPRC5D in a sample collected from the subject using at least one of the above-described chimeric antigen receptor, polynucleotide, or expression vector.

Additional aspects and advantages of the present disclosure will be provided at least in part in the following description, or will become apparent at least in part from the following description, or can be learned from practicing of the present disclosure.

### BRIEF DESCRIPTION OF THE DRAWINGS

The above and/or additional aspects and advantages of the present disclosure will become more apparent and readily understanded from the following description of embodiments taken in conjunction with the accompanying drawings.

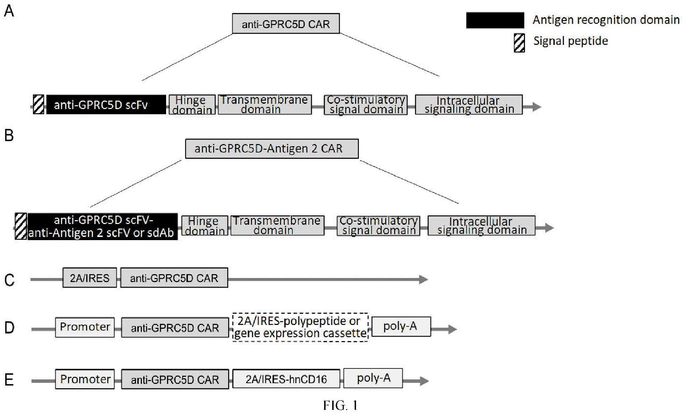

FIG. 1 shows an expression cassette encoding an anti-GPRC5D CAR polypeptide according to an embodiment of the present disclosure, in which FIG. 1A to FIG. 1B show two schematic diagrams of the anti-GPRC5D CAR polypeptide; and FIG. 1C to FIG. 1E show schematic diagrams of several expression cassettes including a polynucleotide encoding the anti-GPRC5D CAR polypeptide.

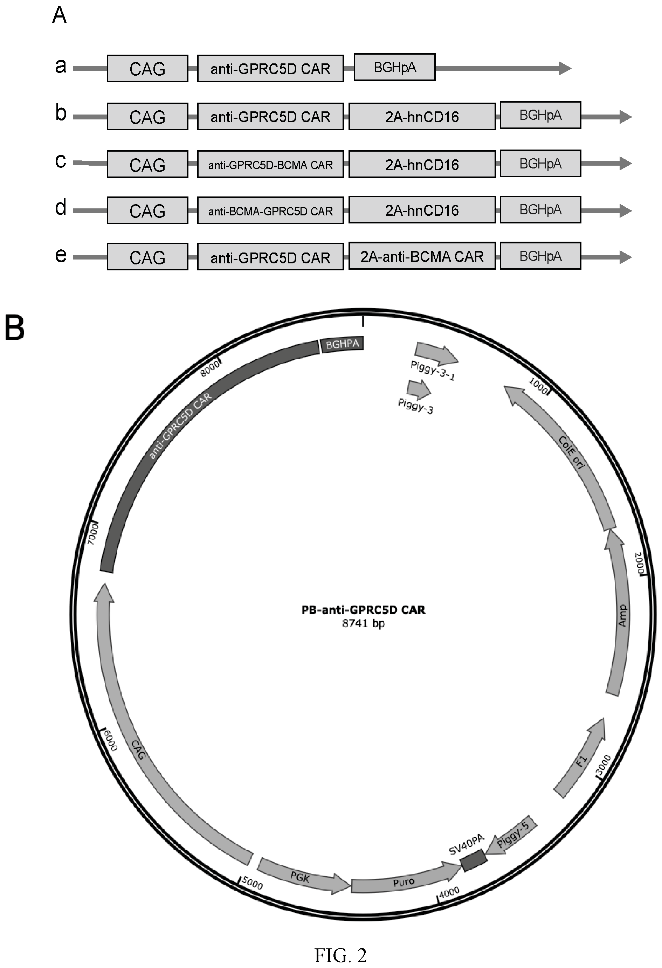

FIG. 2 shows piggyBac-mediated anti-GPRC5D CAR expression according to an embodiment of the present disclosure, in which: FIG. 2A shows several exemplary forms of an anti-GPRC5D CAR polypeptide expression cassette; and FIG. 2B shows a map of a piggyBac plasmid containing the anti-GPRC5D CAR expression cassette.

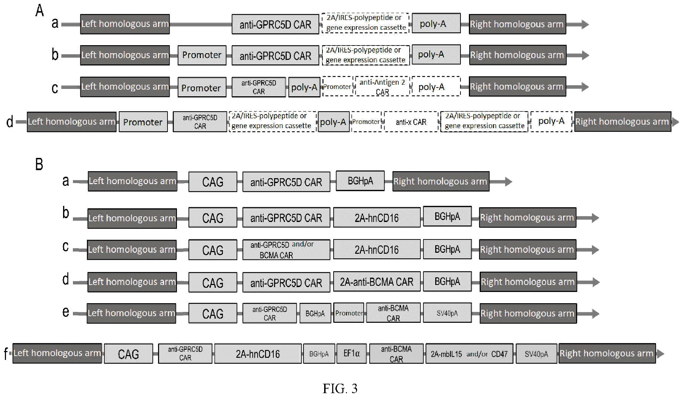

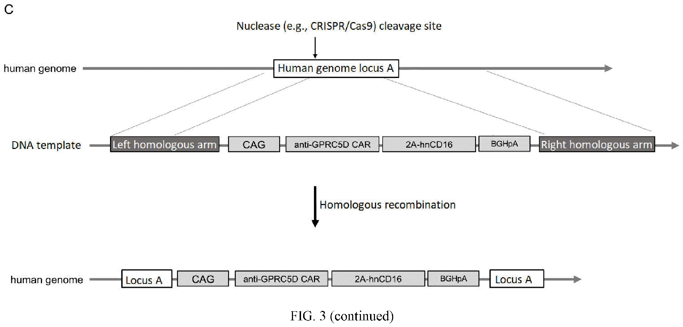

FIG. 3 is a schematic diagram of site-specific integration of a DNA template containing an anti-GPRC5D CAR mediated by homologous recombination according to an embodiment of the present disclosure, in which: FIG. 3A shows schematic diagrams of several universal forms of an expression cassette encoding the anti-GPRC5D CAR polypeptide, with dashed portions indicating optional components that may be present or absent; FIG. 3B shows examples of constituent elements of several DNA templates containing the anti-GPRC5D CAR polypeptide expression cassette; and FIG. 3C shows a schematic diagram of a DNA template expressing anti-GPRC5D CAR and hnCD16, targeted to human genomic locus A (representing any locus in the human genome), as well as homologous recombination.

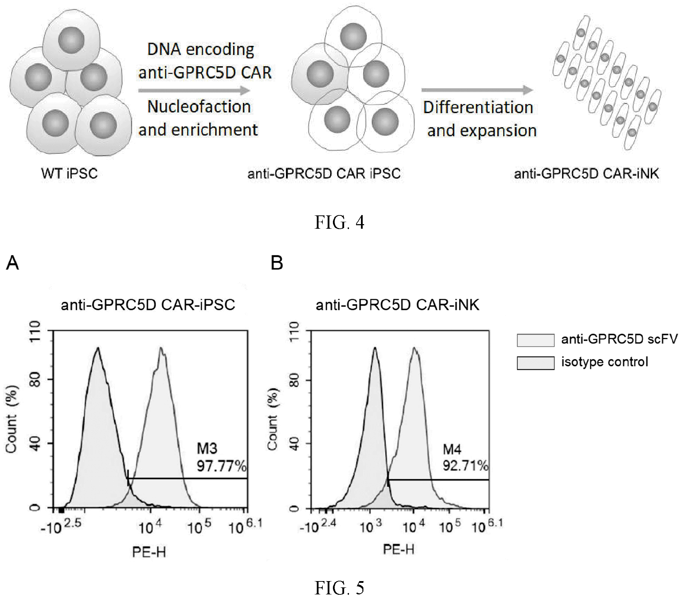

FIG. 4 shows a general workflow for obtaining gene-edited iPSC-derived anti-GPRC5D CAR-iNK cells according to an embodiment of the present disclosure, in which, "*" represents that DNA encoding anti-GPRC5D CAR may be introduced using different tools, such as donor plasmids, piggyBac, dsDNA, ssDNA, and various integrating or non-integrating viruses. Depending on the selected delivery tool, PBase may be selected to assist piggyBac delivery, and nucleases may be selected to form single-strand or double-strand breaks to assist other non-viral delivery methods.
FIG. 5 shows FACS detection results of CAR-positive cell populations in iPSCs and differentiated iNK cells generated via PiggyBac (PB) mediated introduction of anti-GPCR5D CAR according to an embodiment of the present disclosure, in which the proportion of CAR-expressing cells in the tested cell populations was detected using an anti-GPRC5D scFv antibody; the left peak represents the isotype control, and the right peak represents the detected marker.

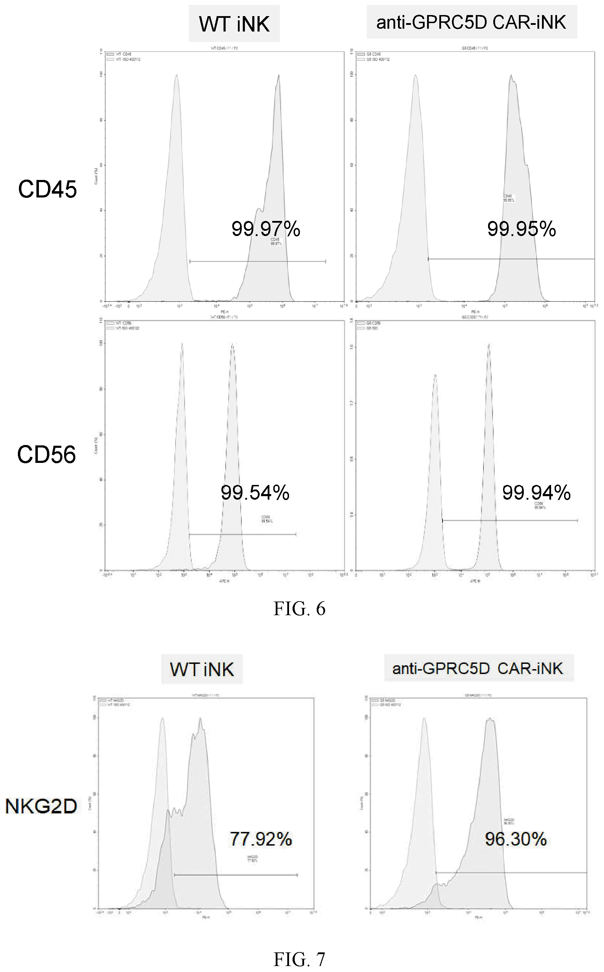

FIG. 6 shows FACS detection results of CD56 and CD45 expression levels in iPSC-derived anti-GPRC5D CAR-iNK cells according to an embodiment of the present disclosure, in which the left peak represents the isotype control, and the right peak represents the detected marker.
FIG. 7 shows FACS detection results of NKG2D expression levels in iPSC-derived anti-GPRC5D CAR-iNK cells according to an embodiment of the present disclosure, in which the left peak represents the isotype control, and the right peak represents the detected marker.

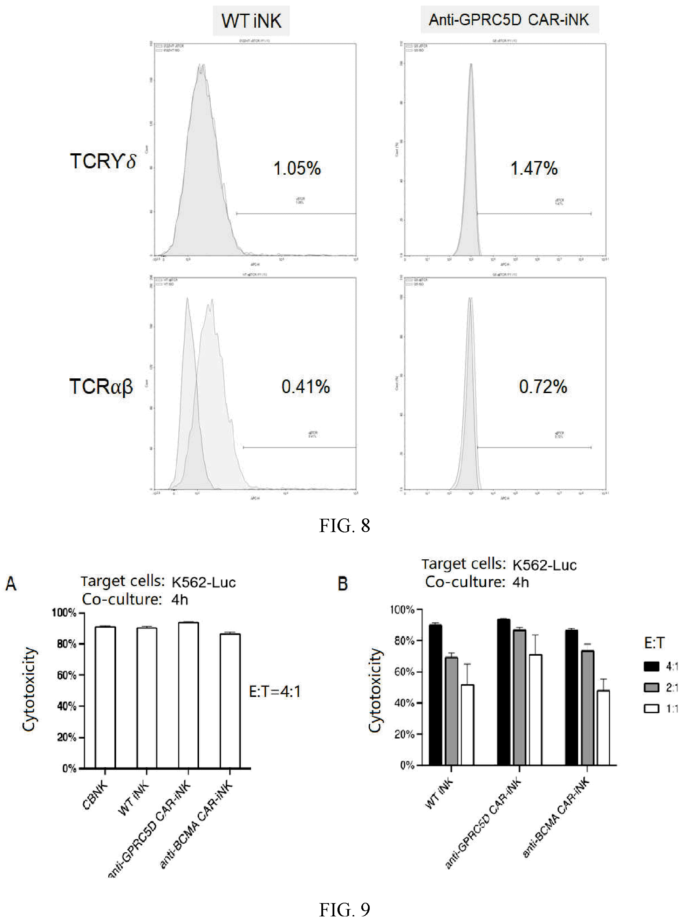

FIG. 8 shows FACS detection results of TCRαβ and TCRΥδ expression levels in iPSC-derived anti-GPRC5D CAR-iNK cells according to an embodiment of the present disclosure, in which the left peak represents the isotype control, and the right peak represents the detected marker.
FIG. 9 shows detection results of killing activities of iPSC-derived anti-GPRC5D or anti-BCMA CAR-iNK cells against K562-Luc cells according to an embodiment of the present disclosure, in which, FIG. 9A shows detection results of killing activities of different NK cells against K562-Luc cells after 4 hours of incubation at an effector-to-target ratio (E:T) of 4:1; and FIG. 9B shows detection results of effects of WT-iNK cells and anti-GPRC5D or anti-BCMA CAR-iNK cells on the killing activity of K562-Luc cells at different effector-to-target ratios.

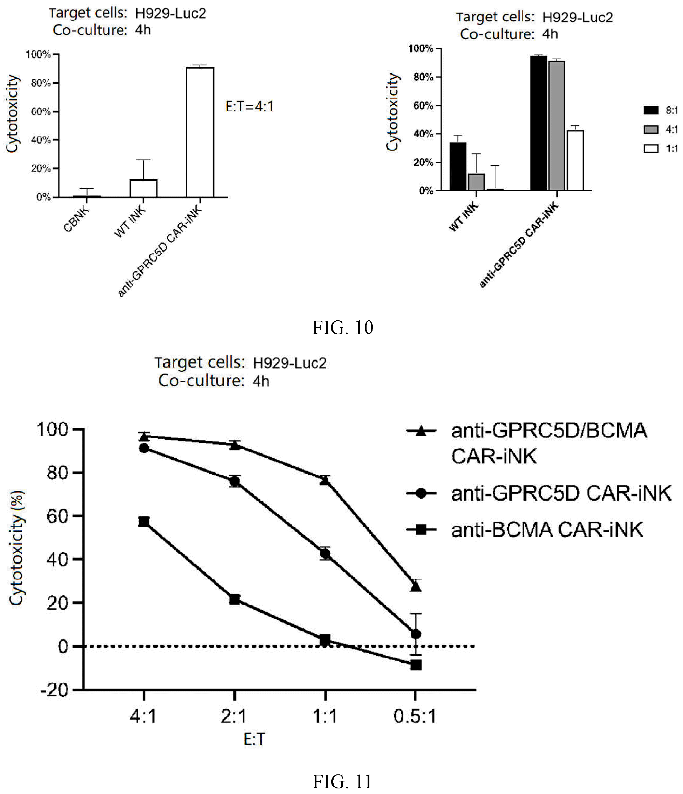

FIG. 10 shows killing activities of iPSC-derived anti-GPRC5D cells against H929-Luc2 cells according to an embodiment of the present disclosure, in which, the left panel shows detection results of killing activities of different NK cells against H929-Luc2 cells after 4 hours of incubation at an effector-to-target ratio of 4:1, and the right panel shows detection results of killing activities of WT-iNK cells and anti-GPRC5D or anti-BCMA CAR-NK cells against H929-Luc2 cells at different effector-to-target ratios.
FIG. 11 shows detection results of killing activities of iPSC-derived anti-GPRC5D/BCMA CAR-NK cells, anti-GPRC5D CAR-NK cells, and anti-BCMA CAR-NK cells against H929-Luc2 cells at different effector-to-target ratios according to an embodiment of the present disclosure, in which the extracellular domain of anti-GPRC5D/BCMA CAR-NK cells contains antibody sequences that recognize both GPRC5D and BCMA.

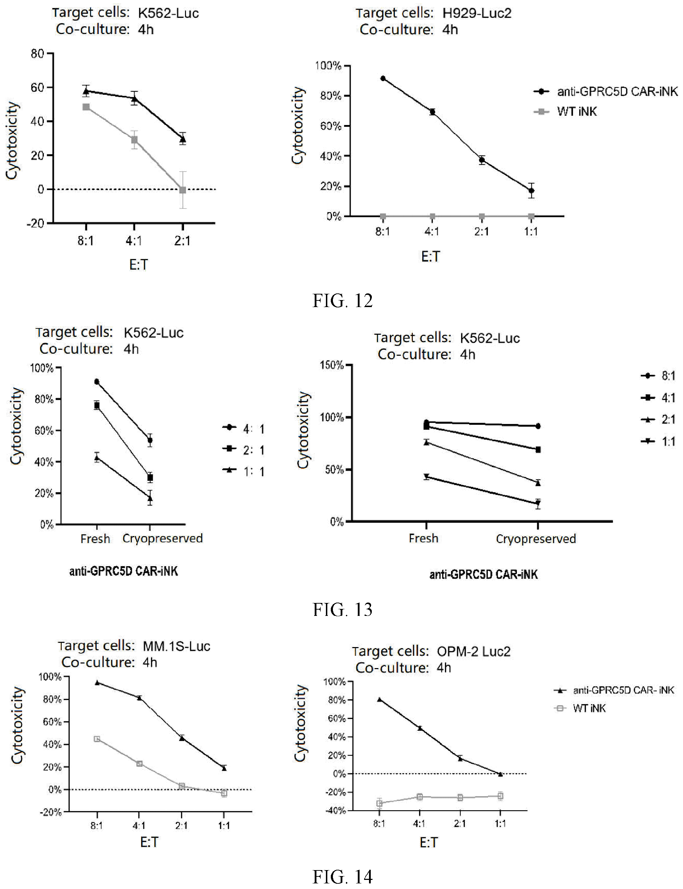

FIG. 12 shows detection results comparing killing activities of cryopreserved iPSC-derived WT-NK cells and anti-GPRC5D CAR-NK cells against K562-Luc cells and H929-Luc2 cells according to an embodiment of the present disclosure.
FIG. 13 shows detection results of changes in killing activities of fresh and cryopreserved anti-GPRC5D CAR-NK cells against K562-Luc and H929-Luc2 cells according to an embodiment of the present disclosure.
FIG. 14 shows detection results of killing activities of cryopreserved anti-GPRC5D CAR-NK cells and WT-NK cells against MM.1S-Luc cells and OPM-2 Luc2 cells at different effector-to-target ratios according to an embodiment of the present disclosure.

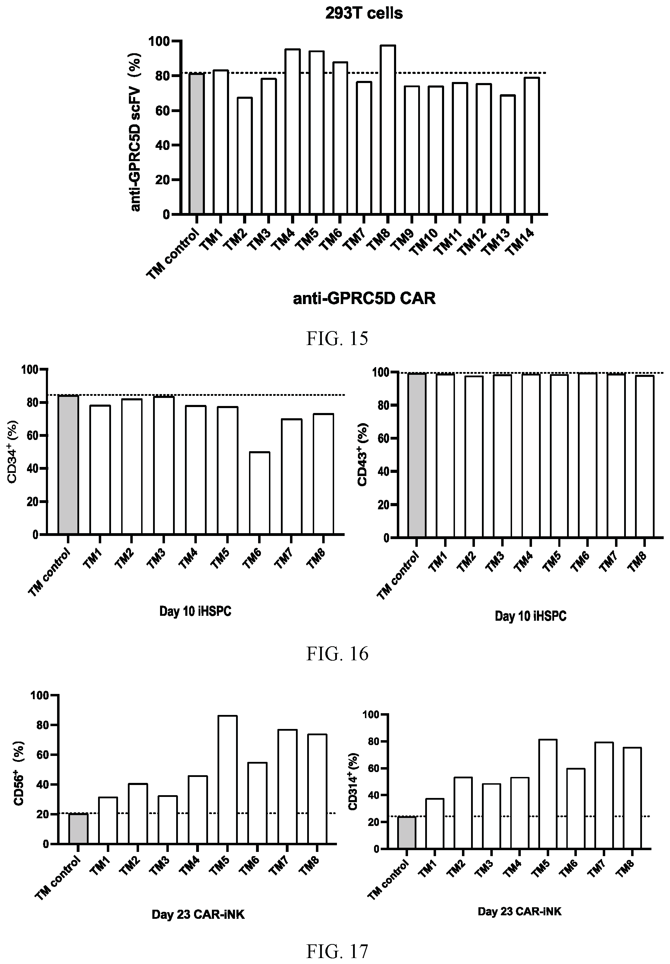

FIG. 15 shows detection results of proportions of CAR-expressing 293T cells using antibodies against anti-GPRC5D scFv after transfection with CAR molecules loaded with anti-GPRC5D scFv (TM control) or variants thereof (TM1 to TM8) according to an embodiment of the present disclosure, in which CAR expression was detected using antibodies against anti-GPRC5D scFv.
FIG. 16 shows flow cytometry detection results of CD34 and CD43 expression levels on day 10 of differentiation of iPSCs into iHSPCs, wherein the iPSCs express CAR molecules loaded with anti-GPRC5D scFv (TM control) or variants thereof (TM1 to TM8) according to an embodiment of the present disclosure, in which the left and right panels show expression percentages of CD34 and CD43 in the iHSPCs differentiated from each group, respectively.
FIG. 17 shows CD56 and CD314 expression levels on day 23 of differentiation of iPSCs into CAR-NK cells, wherein the iPSCs express CAR molecules loaded with anti-GPRC5D scFv (TM control) or variants thereof (TM1 to TM8) according to an embodiment of the present disclosure, in which the left and right panels show expression percentages of CD56 and CD314 in the CAR-NK cells differentiated from each group, respectively.

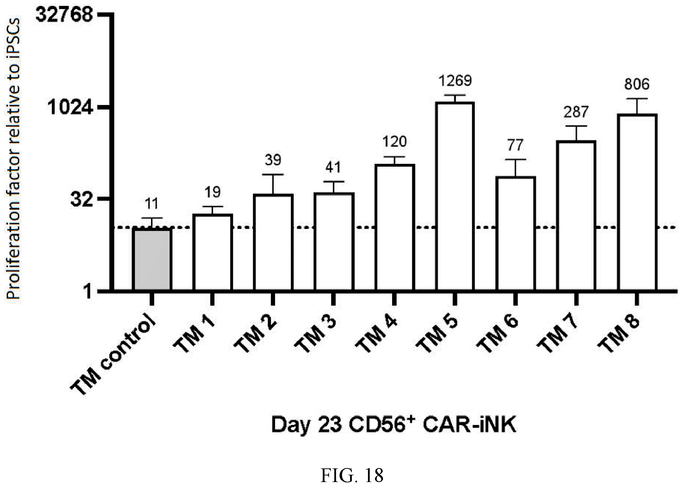

FIG. 18 shows results of fold expansion of CD56⁺ CAR-iNK cell population on day 23 of differentiation of iPSCs into CAR-NK cells relative to the initial iPSC population, wherein the iPSCs express CAR molecules loaded with anti-GPRC5D scFv (TM control) or variants thereof (TM1 to TM8) according to an embodiment of the present disclosure.

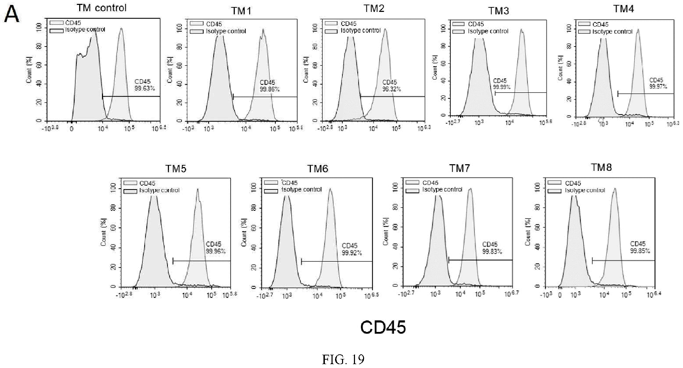

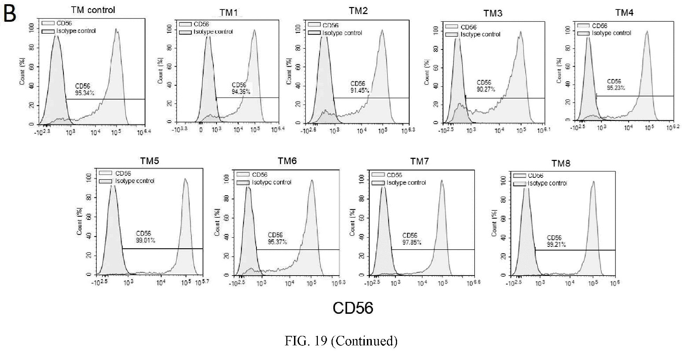

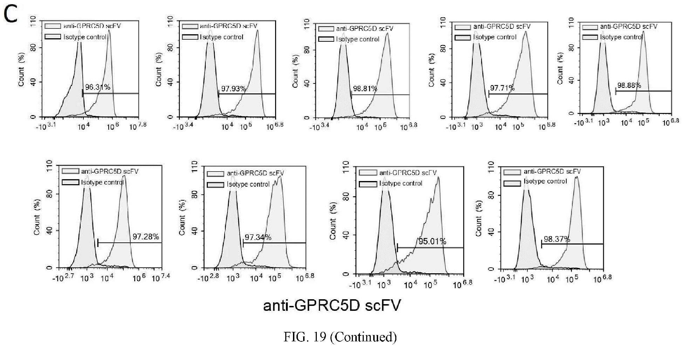

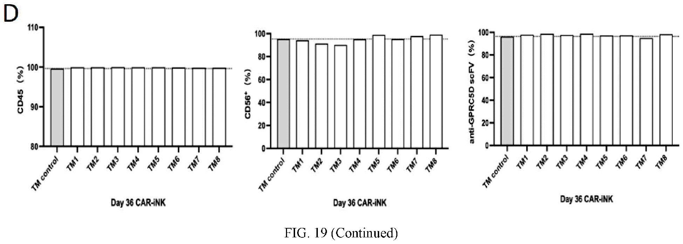

FIG. 19 shows expression of CD45, CD56, and anti-GPRC5D scFv in CAR-NK cells (day 36 CAR-NK cells) after an additional 13 days of expansion, wherein the CAR-NK cells express CAR molecules loaded with anti-GPRC5D scFv (TM control) or variants thereof (TM1 to TM8) according to an embodiment of the present disclosure, in which FIG. 19A, 19B, and 19C show flow cytometry detection results of expression percentages of CD45, CD56, and anti-GPRC5D scFv, respectively, where the left peak represents the isotype control, and the right peak represents the detected marker (CD45, CD56, and anti-GPRC5D scFv), and FIG. 19D shows statistical analysis of the expression percentages of CD45, CD56, and anti-GPRC5D scFv detected by flow cytometry.

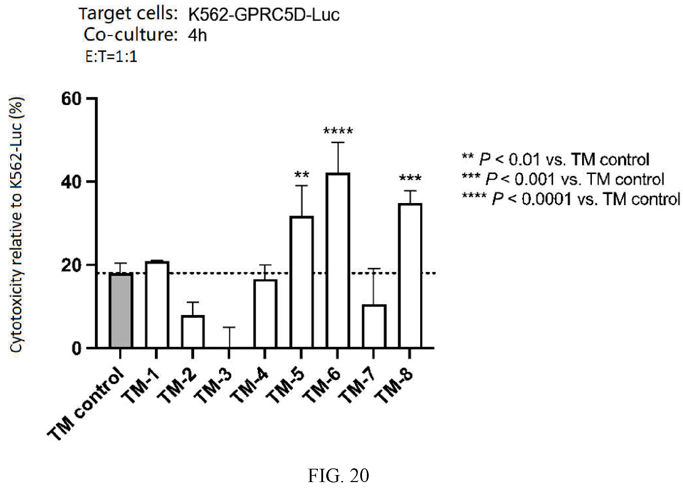

FIG. 20 shows detection results of antigen-dependent specific killing activities of CAR-NK cells expressing CAR molecules loaded with anti-GPRC5D scFv (TM control) and variants thereof (TM 1 to TM 8) against K562-GPRC5D-Luc cells according to an embodiment of the present disclosure, in which statistical analysis is performed using one-way ANOVA followed by Dunnett's multiple comparison test (GraphPad Prism 9.5.0 (525)).

### DETAILED DESCRIPTION OF THE EMBODIMENTS

Embodiments of the present disclosure will be described in detail below. The embodiments described below are exemplary and are intended only to explain the present disclosure, and they should not be construed as limiting the present disclosure.

It should be noted that, the terms "first" and "second" are only used for descriptive purposes, and cannot be understood as indicating or implying relative importance or implicitly indicating the number of indicated technical features. Therefore, the feature associated with "first" and "second" may include one or more this feature distinctly or implicitly. Further, in the description of the present disclosure, unless otherwise specified, "more" or "a plurality of" means two or more.

In the present disclosure, endpoints and any value of the ranges shall not be limited to the exact range or value, and those ranges or values should be understood to include values close to those ranges or values. For numerical ranges, endpoints of respective ranges, an endpoint and individual point value of respective ranges, and individual point values can be combined with each other to obtain one or more new numerical ranges, which should be deemed to be specifically disclosed herein.

To make the present disclosure more easily understood, certain technical and scientific terms are specifically defined below. Unless otherwise clearly defined, all other technical and scientific terms used herein have the meanings commonly understood by those skilled in the art to which the present disclosure belongs.

As used in the present disclosure, the terms "comprising", "containing" or "including" are open-ended expressions and are intended to cover the components explicitly mentioned as well as additional elements not expressly recited.

As used in the present disclosure, the terms "optionally," "optional," or "option" mean that the subsequently described event or circumstance may or may not occur, and that the description includes both instances where the event or circumstance occurs and where it does not.

### Terms

"scFv" stands for "single-chain fragment variable" and refers to a genetically engineered fusion protein, which is a small molecule composed of the variable region of the heavy chain and the variable region of the light chain of the antibody linked by a peptide chain.

The "antibody" includes polyclonal and monoclonal antibodies, as well as intact antibodies and functional (antigen-binding) antibody fragments. Such fragments include fragment antigen-binding (Fab) fragments, F(ab')2 fragments, Fab' fragments, Fv fragments, recombinant IgG (rIgG) fragments, variable heavy chain (VH) regions capable of specifically binding to antigens, and single-chain antibody fragments including single-chain variable fragments (scFv) and single-domain antibody fragments (e.g., sdAb, sdFv, nanobodies). Examples also include bispecific antibodies, diabodies, triabodies, tetrabodies, tandem di-scFv, and tandem tri-scFv. Unless otherwise specified, the term "antibody" should be understood to include its functional antibody fragment.

The "antibody fragment" refers to a molecule, other than an intact antibody, that contains a portion of an intact antibody capable of binding to an antigen. Examples of antibody fragments include, but are not limited to, Fv, Fab, Fab', Fab'-SH, F(ab')2; diabodies; linear antibodies; variable heavy chain regions, single-chain antibody molecules (such as scFv), and single-domain antibodies; as well as multispecific antibodies formed from antibody fragments. In certain embodiments, the antibody is a single-chain antibody fragment containing a variable heavy chain region and/or a variable light chain region, such as scFv.

As used herein, the "heavy chain variable region" refers to a region of an antibody molecule that contains at least one complementarity-determining region (CDR) of the heavy chain variable domain of the antibody. The heavy chain variable region may contain one, two, or three CDRs of the heavy chain of the antibody.

As used herein, the term "single-domain antibody (abbreviated as sdAb)" refers to a naturally occurring heavy chain antibody found in camel/alpaca blood that lacks the light chain and consists only of the heavy chain (H chain) with a relatively high molecular weight. The amino acid sequence at the amino-terminus (N-terminus) of the peptide chain varies considerably, which is referred to as the variable region (V region), while the carboxyl-terminus (C-terminus) is relatively stable and varies little, which is referred to as the constant region (C region). The V region of the H chain is called VH. Within the variable region, certain regions exhibit a higher degree of variation in amino acid composition and sequence arrangement, which are called hypervariable regions (HVRs). These hypervariable regions are the sites where antigens bind to antibodies and are therefore also called complementarity-determining regions (CDRs). The heavy chain variable region includes three CDRs, CDR1 and CDR3 are slightly longer than in humans, and CDR3 protrudes outward in the tertiary structure. Therefore, it is speculated that the single-domain antibody has higher antigen-binding specificity and affinity than the conventional antibody. In some embodiments, the single-domain antibody is a human single-domain antibody or an alpaca single-domain antibody with or without humanization. The variable domain of a single-domain antibody exhibits the same general structure of a relatively conserved framework region (FR) linked by three hypervariable regions or CDRs. From the N-terminus to the C-terminus, the heavy chain contains domains FR1, CDR1, FR2, CDR2, FR3, CDR3, and FR4. The amino acid assignment of each domain conforms to the definition of Kabat Sequences of Proteins of Immunological Interest (National Institutes of Health, Bethesda, Md., (1987 and 1991); or Chothia and Lesk, Journal of Molecular Biology, 196:901-917, 1987; Chothia et al., Nature, 342:878-883, 1989).

The "hypervariable region of an antibody" refers to the CDR amino acid residues of the antibody responsible for antigen binding. The hypervariable region contains amino acid residues from the CDRs, such as 31-35 (H1), 50-65 (H2), and 95-102 (H3) in the heavy chain variable domain (as described in Kabat et al., Sequences of Proteins of Immunological Interest, 5th Edition, Public Health Service, National Institutes of Health, Bethesda, MD (1991)); and/or residues from the hypervariable loop, such as 26-32 (H1), 53-55 (H2), and 96-101 (H3) in the heavy chain variable domain (as described in Chothia et al., Journal of Molecular Biology 196:901-917 (1987)).

A "monoclonal antibody" refers to an antibody obtained from a substantially homogeneous population of antibodies, and all antibodies in the mixture have a single amino acid sequence from a single clone. Monoclonal antibodies are typically highly specific and target a single antigenic site or epitope. In contrast, polyclonal antibody formulations usually include a mixture of antibodies with different amino acid sequences that target the same or different determinants (epitopes). In addition to their specificity, monoclonal antibodies have the advantages of being synthesized in homologous cultures, free from contamination by other immunoglobulins with different specificities and characteristics.

As used herein, the term "hinge domain (Hinge)" refers to a region between the CH1 region and the CH2 region of the antibody heavy chain. This region includes inter-heavy-chain disulfide bonds, is rich in proline, does not form α-helix, and is susceptible to extension and a certain degree of distortion, facilitating complementary binding between the antigen-binding site of the antibody and the antigen epitope. In the present disclosure, the antigen-binding domain is linked to the transmembrane domain via the hinge domain.

In the present disclosure, the term "antigen" specifically refers to a marker on the surface of tumor cells.

The term "chimeric antigen receptor", abbreviated as CAR, refers to an artificial receptor molecule manufactured using genetic engineering technology, which includes an antigen-binding domain (usually from a gene fragment of the variable region of a single-chain antibody, scFV; or from a single-domain antibody), a hinge domain, a transmembrane domain, a co-stimulatory signal domain, and an intracellular signaling domain.

As described herein, the term "reduced expression" of a gene refers to the elimination or reduction in the expression of one or more gene products encoded by a gene in cells compared with the expression level of the mRNA or protein of the gene under normal conditions. In some cases, this effect on gene expression is temporary or reversible. For example, gene expression is inhibited through small molecule compounds, nucleic acids such as RNAi, siRNA, shRNA, or specific proteins. In some cases, this effect on gene expression is permanent. For example, base insertions or deletions are introduced into the coding region of a target gene through gene editing to induce frameshift mutations. Alternatively, a DNA sequence is inserted into the coding or non-coding region of the target gene through homologous recombination, which results in frameshift mutations of the gene, premature termination of transcription and/or translation, or the translation of a completely distinct polypeptide, enabling permanent inactivation of the gene. The percentage of reduced gene expression caused by different methods may be 100%, 90% or more, 80% or more, 70% or more, 60% or more, 50% or more, 40% or more, 30% or more, or 20% or more.

As used herein, the term "inactivation" refers to loss of function or loss of protein function.

As used herein, the term "nucleic acid" is used interchangeably with the term "polynucleotide", refers to deoxyribonucleotides or ribonucleotides and their polymers in single-stranded or double-stranded form, and encompasses nucleic acids containing known nucleotide analogs or modified backbone residues or linkages. The nucleic acids are synthetic, naturally occurring, or non-naturally occurring, have binding properties similar to those of a reference nucleic acid, and are metabolized in a manner similar to that of a reference nucleotide. Examples of such analogs include, but are not limited to, thiophosphates, aminophosphates, methylphosphonates, chiral-methylphosphonates, 2-O-methyl ribonucleotides, and peptide-nucleic acids (PNAs). A nucleic acid encoding a polypeptide or a fusion protein refers to one or more nucleic acid molecules that encode a polypeptide or fusion protein, including one or more nucleic acid molecules in a single vector or separate vectors, and one or more nucleic acid molecules existing at one or more locations in a host cell. Unless otherwise specified, a specific nucleic acid sequence also implicitly encompasses its conservatively modified variants (e.g., degenerate codon substitutions), complementary sequences, and explicitly stated sequences.

As used herein, the term "vector" refers to a delivery vehicle into which a genetic element (e.g., the above-described nucleic acid molecule) can be operably inserted to enable the expression of the genetic element, for example, to produce a protein, RNA, or DNA encoded by the genetic element, or to replicate the genetic element. The vector can be used to transform, transduce, or transfect host cells, allowing the genetic elements they carry to be expressed in the host cells. For example, the vector includes plasmids, phagemids, cosmids, artificial chromosomes such as yeast artificial chromosomes (YAC), bacterial artificial chromosomes (BAC), or P1-derived artificial chromosomes (PAC), bacteriophages such as λ phage or M13 phage, and animal viruses. The vector may contain a variety of elements that control expression, including promoter sequences, transcription initiation sequences, enhancer sequences, selection elements, and reporter genes. In addition, the vector may contain an origin of replication. The vector may also include components that facilitate its entry into the cell, including but not limited to viral particles, liposomes, or protein coats. The vector may be an expression vector or a cloning vector. In some embodiments, the vector (e.g., the expression vector) in the present disclosure contains a nucleic acid sequence encoding a fusion protein in the present disclosure, at least one promoter operably linked to the nucleic acid sequence (e.g., SV40, CMV, EF-1α), and at least one selectable marker.

As used herein, the term "cell" refers to a eukaryotic cell, particularly a mammalian cell, such as a human cell.

As used herein, the term "immune cell" in the present disclosure refers to a cell from a human. More specifically, the immune cells in the present disclosure may be obtained via directed differentiation of pluripotent stem cells. Depending on the different differentiation conditions provided, these immune cells may be T cells, NK cells, and macrophages. The immune cells of the present disclosure may also be from human hematopoietic stem cells. These hematopoietic stem cells may be isolated from peripheral blood, umbilical cord blood, and placental blood, or differentiated from pluripotent stem cells. Depending on the different differentiation conditions provided, these immune cells may be T cells, NK cells, and macrophages. The immune cells in the present disclosure may also be isolated from human blood, bone marrow, lymph, lymphoid organs, or specific tissues (including but not limited to tumor tissues, abdominal cavity, various visceral organs such as liver and lungs, muscles, etc.). For example, the immune cells include cells of innate or adaptive immunity, such as bone marrow or lymphocytes, typically T cells, NK cells, macrophages, etc. Preferably, according to the present disclosure, the immune cells include T cells, NK cells, and macrophages. The immune cells of the present disclosure may also be differentiated from immune progenitor cells, such as lymphoid progenitor cells.

As used herein, the term "pluripotent stem cells" generally refer to human embryonic stem cells (ESCs) or induced pluripotent stem cells (iPSCs). Human ESCs are isolated from blastocysts during the development of human zygotes, or from embryos obtained through nuclear transfer (embryonic stem cells within 14 days after fertilization and not subjected to in vivo development). Human iPSCs may be obtained by reprogramming human cells through the overexpression of specific transcription factors (such as OCT4, SOX2, KLF4, MYC). Both human ESCs and iPSCs express OCT4, NANOG, Tra-1-60, and SSEA-4. A significant characteristic of pluripotent stem cells that do not express SSEA-1 is their ability to differentiate into tissues or cells of three germ layers in vitro or in vivo.

As used herein, the term "hematopoietic stem cells" refers to CD34⁺ cells. These cells can be isolated from peripheral blood, umbilical cord blood, placental blood, bone marrow, spleen, lymph nodes, or other tissues, or differentiated from pluripotent stem cells.

As used herein, the term "progenitor cells" are cells that can differentiate into various types of immune cells, such as myeloid stem cells or lymphoid stem cells. Further, the term "progenitor cells" also refer to erythroid stem cells, granulocyte stem cells, monocyte stem cells, megakaryocyte stem cells, precursor B cells, precursor T cells, NK progenitor cells, and macrophage progenitor cells.

As used herein, the term "NK cells" refer to natural killer cells, which are generally CD56⁺CD45⁺ double-positive and express various activation receptors, such as NKG2D, NKp44, and NKp46. NK cells can be differentiated from pluripotent stem cells (such as iPSCs), isolated from peripheral blood, differentiated from CD34⁺ cells (such as umbilical cord blood or placental blood), or obtained from tumor cell lines (such as NK92).

As used herein, the term "T cells" refers to CD4⁺ or CD8⁺ T cells, double-negative T cells (CD3⁺CD4⁻CD8⁻),γδ T cells (expressing γδ TCR), NKT cells, or NKT-like cells (CD3⁺CD56⁺). The cells may be naive T cells (TN cells), T memory stem cells (TSCM cells), memory T cells (TCM cells), regulatory T cells (Treg cells), TCR-T cells, tumor-infiltrating lymphocytes (TILs), effector memory T cells (TEM cells), or combinations thereof. "CBNK" refers to NK cells from umbilical cord blood, and "WT iNK" refers to NK cells from wild-type iPSCs. "Anti-BCMA CAR-iNK" refers to NK cells from iPSCs that express anti-BCMA CAR. "Anti-GPRCSD CAR-iNK" refers to NK cells from iPSCs that express anti-GPRC5D CAR.

As used herein, the term "composition" refers to a form in which the biological activity of the active ingredient is effective and which does not contain any additional ingredients that exhibit unacceptable toxicity to the subject to whom the composition is to be administered.

As used herein, the term "treatment" refers to the temporary or permanent, partial or complete elimination, reduction, inhibition, or improvement of clinical symptoms, manifestations, or progression of an event, a disease, or a symptom.

As used herein, the term "diagnosis" refers to the identification, revelation, confirmation, and/or definition of the positioning of the pathogenic process of a pathological state, disease, or condition. In some specific embodiments, the pharmaceutical composition of the present disclosure, when administered to a subject or exposed to a sample from a subject, facilitates the diagnosis of cancer, tumor formation, or condition.

As used herein, an autoimmune disease is a disease caused by the immune system mistakenly attacking the body's own tissues. More than 80 types of autoimmune diseases are currently known. These diseases can be classified into systemic autoimmune diseases and organ-specific autoimmune diseases. The autoimmune diseases include, but are not limited to, at least one of systemic lupus erythematosus, rheumatoid arthritis, systemic sclerosis, arthritis syndrome, autoimmune anemia, rheumatoid heart disease, Sjögren's syndrome, systemic vasculitis, pemphigus, dermatomyositis, autoimmune hemolytic anemia, thyroid autoimmune disease, Hashimoto's thyroiditis, type 1 diabetes, and multiple sclerosis.

As used herein, a viral infectious disease refers to a disease caused by viral infection, which is defined as a process in which a virus invades the body through various routes and proliferates in a susceptible host cell. The viral infectious disease includes, but is not limited to, EBV infectious diseases.

In many embodiments, the terms "individual," "subject," and "patient" are used interchangeably, regardless of whether the subject has undergone or is currently undergoing any form of treatment. As used herein, the terms "individual," "subject," or "patient" refer to a mammalian individual, subject, or patient. Unless otherwise indicated, the terms "individual," "patient," or "subject" are used interchangeably herein. Examples include, but are not limited to, humans, monkeys, dogs, cats, mice, rats, cattle, horses, camels, poultry, goats, and sheep. In some embodiments, the subject is a human. In some embodiments, the subject is a person suspected of suffering from cancer, an autoimmune disease or condition, and/or an infection.

As used herein, the term "tumor antigen" is a general term for protein and polypeptide molecules that are newly emerged and highly expressed during malignant transformation of cells. According to the relationship between tumor antigens and tumors, tumor antigens are divided into two main categories: "tumor-specific antigens" (TSA) and "tumor-associated antigens" (TAA). TSA refers to antigens specific to tumor cells and not present on normal tissue cells. TAA refers to antigens that are not specific to tumor cells and can also exist on normal tissue cells, especially embryonic tissues. Therefore, TAA only exhibits quantitative changes between tumor cells and normal tissues. In terms of the origin or characteristics of antigens, TAA generally refers to proteins expressed in tumors induced by chemical and physical carcinogenic factors, proteins expressed in tumors induced by viruses, abnormal proteins encoded by oncogenes and mutant tumor suppressor genes, or proteins expressed after the activation of quiescent genes. TAA generally refers to embryonic proteins, differentiation proteins, proteins encoded by highly expressed oncogenes, or excessively or abnormally expressed glycolipids and glycoproteins (Principles of Immunology (4th Edition), edited by Zhou Guangyan, Beijing: Science Press, 2018.1; ISBN 978-7-03-053131-5; p339).

### Chimeric Antigen Receptor

The present disclosure provides a chimeric antigen receptor. The chimeric antigen receptor includes: an extracellular domain, capable of binding to a tumor antigen, an inflammatory factor, or a pathogen antigen; a transmembrane domain, linked to the extracellular domain, the transmembrane domain being from a type I transmembrane protein or an artificially synthesized transmembrane protein with a hydrophobic helix; and an intracellular domain, linked to the transmembrane domain. The amino acid sequence of the transmembrane domain is SNLFVASWIAVMX₈IFX₉IGX₁₀AX₁₁AX₁₂X₁₃X₁₄X₁₅X₁₆X₁₇X₁₈X₁₉PS. X₈ is selected from I or V, X₉ is selected from R or H, X₁₀ is selected from M or L, X₁₁ is selected from V or I, X₁₂ is selected from I or V, X₁₃ is selected from S or F, X₁₄ is selected from C, R, or H, X₁₅ is selected from C, T, or V, X₁₆ is selected from V, L, or absent, X₁₇ is selected from F or H, X₁₈ is selected from F or Y, X₁₉ is selected from L or absent, and X₁₆ and X₁₉ are not present simultaneously and X₁₆ and X₁₉ are not absent simultaneously. The tumor antigen is a tumor-specific antigen or a tumor-associated antigen.

The present disclosure further optimizes the amino acid sequence of the transmembrane domain. When the transmembrane domain of the chimeric antigen receptor has the amino acid sequence as set forth in the above-described general formula, the engineered immune cell containing the chimeric antigen receptor exhibits an enhanced ability to kill a target cell (a cell which the extracellular domain targets and binds to).

According to an embodiment of the present disclosure, the transmembrane domain has the amino acid sequence as set forth in any one of SEQ ID NO: 19 to SEQ ID NO: 23 and SEQ ID NO: 27 to SEQ ID NO: 29.

The present disclosure provides a chimeric antigen receptor (CAR). The CAR includes: an extracellular domain, capable of binding to a tumor antigen; a transmembrane domain, linked to the extracellular domain, the transmembrane domain being from a type I transmembrane protein or an artificially synthesized transmembrane protein with a hydrophobic helix; and an intracellular domain, linked to the transmembrane domain. The amino acid sequence of the transmembrane domain is SNLFVASWIAVMX₁IFX₂IGMAX₃AIX₄X₅CX₆FFX₇PS. X₁ is selected from I or V, X₂ is selected from R or H, X₃ is selected from V or I, X₄ is selected from S or F, X₅ is selected from C or R, X₆ is selected from L or absent, and X₇ is selected from L or absent. The tumor antigen is a tumor-specific antigen or a tumor-associated antigen.

According to an embodiment of the present disclosure, the transmembrane domain has the amino acid sequence as set forth in any one of SEQ ID NO: 19 to SEQ ID NO: 21 and SEQ ID NO: 23.

The present disclosure provides a chimeric antigen receptor. The chimeric antigen receptor includes: an extracellular domain, capable of binding to a tumor antigen, an inflammatory factor, or a pathogen antigen; a transmembrane domain, linked to the extracellular domain, the transmembrane domain being from a type I transmembrane protein or an artificially synthesized transmembrane protein with a hydrophobic helix; and an intracellular domain, linked to the transmembrane domain. The amino acid sequence of the transmembrane domain is SNLFVASWIAVMIIFX₂₀IGX₂₁AX₂₂AX₂₃SX₂₄X₂₅X₂₆X₂₇X₂₈PS. X₂₀ is selected from R or H, X₂₁ is selected from M or L, X₂₂ is selected from V or I, X₂₃ is selected from V or I, X₂₄ is selected from C, R, or H, X₂₅ is selected from C, T, or V, X₂₆ is selected from L or V, X₂₇ is selected from F or H, and X₂₈ is selected from F or Y. The tumor antigen is a tumor-specific antigen or a tumor-associated antigen.

The present disclosure further optimizes the amino acid sequence of the transmembrane domain. When the transmembrane domain of the chimeric antigen receptor has the amino acid sequence as set forth in the above-described general formula, the engineered immune cell containing the chimeric antigen receptor exhibits an enhanced ability to kill a target cell (a cell which the extracellular domain targets and binds to).

According to an embodiment of the present disclosure, the transmembrane domain has the amino acid sequence as set forth in any one of SEQ ID NO: 22 and SEQ ID NO: 27 to SEQ ID NO: 29.

According to an embodiment of the present disclosure, the above-described chimeric antigen receptors may further include at least one of the following additional technical features:

According to an embodiment of the present disclosure, the extracellular domain includes an antibody or antigen-binding fragment thereof capable of specifically recognizing a tumor antigen, an inflammatory factor, or a pathogen antigen.

According to an embodiment of the present disclosure, the extracellular domain includes an antibody or antigen-binding fragment thereof capable of specifically recognizing at least one selected from the group consisting of: CD19, BCMA, GPRC5D, CD123, CD138, CD20, CD22, CD30, CD33, CD38, CD4, CD7, CLL-1, CD10, CD34, CS1, CD16, CD5, IL-1-RAP, ITGB7, k-IgG, TACI, TRBC1, MUC1, NKG2DL, PD-L1, CD133, CD117, LeY, CD70, ROR1, CD147, CD171, CD267, AXL, B7-H3, CD44v6, CEA, CAIX, CLDN18.2, CLDN6, DLL3, DR5, EGFR, EpCAM, ErbB, FAP, FBP, FRα, GD2, gp100, HER2, IL-13Rα2, LFA1, LMP1, MAGE-A3, MAGE-A1, MAGE-A4, MART1, MET, MG7, MMP2, MSLN, MUC1, MUC16, MUC16ecto, NECTIN4, NKG2D, PMEL, PSCA, PSMA, ROR1, ROR2, TM4SF1, TnMUC1, VEGFR2, CLD18, and GPC3.

According to an embodiment of the present disclosure, the extracellular domain further includes an antibody or antigen-binding fragment thereof capable of specifically recognizing a pathogen antigen. The pathogen includes EBV.

According to an embodiment of the present disclosure, the extracellular domain further includes an antibody or antigen-binding fragment thereof capable of specifically recognizing an inflammatory factor. The inflammatory factor includes at least one selected from the group consisting of: IL-1, TNF-α, IFN-γ, IL-12, IL-18, GMCSF, IL-6, IL-8, IL-17, CXCL1, CXCL2, CXCL9, CXCL10, CXCL11, CXCL16, and CCL2-2.

According to an embodiment of the present disclosure, the extracellular domain includes an antibody or antigen-binding fragment thereof capable of specifically recognizing GPRC5D.

According to an embodiment of the present disclosure, the antibody or the antigen-binding fragment capable of specifically recognizing GPRC5D includes at least one CDR sequence selected from the following or an amino acid sequence having at least 90% identity thereto:
heavy chain variable region CDR sequences: SEQ ID NO: 1 to SEQ ID NO:3; and
light chain variable region CDR sequences: SEQ ID NO: 4, the amino acid sequence SAS, and SEQ ID NO: 5.

According to a preferred embodiment of the present disclosure, the antibody or the antigen-binding fragment capable of specifically recognizing GPRC5D in the present disclosure includes:
heavy chain variable region CDR1, CDR2, and CDR3 sequences as set forth in SEQ ID NO: 1, SEQ ID NO: 2, and SEQ ID NO: 3, respectively, or amino acid sequences having at least 90% identity thereto, and light chain variable region CDR1, CDR2, and CDR3 sequences as set forth in SEQ ID NO: 4, the amino acid sequence SAS, and SEQ ID NO: 5, respectively, or amino acid sequences having at least 90% identity thereto.

It should be noted that, any form of antibody or antibody fragment including the above-described heavy chain variable region CDR1, CDR2, CDR3 sequences and light chain variable region CDR1, CDR2, CDR3 sequences is within the scope of protection of the present disclosure, such as IgG, scFv, Fab, and Fab'.

According to a preferred embodiment of the present disclosure, the antibody or the antigen-binding fragment capable of specifically recognizing GPRC5D includes a heavy chain variable region having the amino acid sequence as set forth in SEQ ID NO: 6 and a light chain variable region having the amino acid sequence as set forth in SEQ ID NO: 7.

According to a preferred embodiment of the present disclosure, the antibody is a single-chain antibody having the amino acid sequence as set forth in SEQ ID NO: 8. The GPRC5D single-chain antibody in the present disclosure is also abbreviated as GPRC5D scFV.

According to an embodiment of the present disclosure, the extracellular domain includes a bispecific binding molecule. The bispecific binding molecule includes:
a first binding region, including the antibody or antigen-binding fragment thereof capable of specifically recognizing GPRC5D; and
a second binding region, having an activity of binding to an antigen specifically expressed on lymphocytes.

According to an embodiment of the present disclosure, all the antibodies that bind to antigens specifically expressed on the lymphocytes are included within the scope of the second binding regions of the bispecific binding molecules of the present disclosure.

According to a specific embodiment of the present disclosure, the antigen specifically expressed on the lymphocytes includes at least one selected from the group consisting of BCMA, CD38, CD138, CD3E, and FCGR3A. The antigens specifically expressed on the lymphocytes described in the present disclosure are not limited to the above-described types, but may also be any type of antigens specifically expressed on the lymphocytes known in the art. The antibodies or the antigen-binding fragments thereof capable of specifically recognizing GPRCSD, as well as bispecific binding molecules having the activity of binding to the antigens specifically expressed on lymphocytes are all within the scope of protection of the present disclosure. Preferably, the second binding region may be an intact antibody, a single-chain antibody, or a single-domain antibody.

According to a preferred embodiment of the present disclosure, the second binding region has BCMA-binding activity and includes a single-chain antibody or a single-domain antibody capable of specifically recognizing BCMA.

According to a preferred embodiment of the present disclosure, the second binding region has the amino acid sequence as set forth in SEQ ID NO: 9.

According to a preferred embodiment of the present disclosure, the bispecific binding molecule includes an antibody or antigen-binding fragment thereof capable of specifically recognizing GPRC5D and a single-chain antibody or single-domain antibody capable of specifically recognizing BCMA. The GPRC5D single-chain antibody is linked to the BCMA single-chain antibody or single-domain antibody via a linking peptide, enabling targeted killing of multiple myeloma cells. Preferably, the bispecific binding molecule has the amino acid sequence as set forth in SEQ ID NO: 10.

In the present disclosure, in addition to an extracellular targeting moiety capable of recognizing the GPRC5D protein, the CAR may further include a hinge domain. The hinge domain may be located between the extracellular antigen recognition domain and the transmembrane domain. For example, the hinge domain may include a hinge domain of one or more proteins selected from the group consisting of: CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT. For example, the hinge region may be a hinge region from CD8. For example, the hinge domain may have the amino acid sequence as set forth in SEQ ID NO: 11.

In the present disclosure, the intracellular domain includes a co-stimulatory signal domain and an intracellular signaling domain that are linked via a linking peptide. In addition to an extracellular targeting portion capable of recognizing the GPRC5D protein, the CAR may further include the co-stimulatory signal domain capable of providing a stimulatory signal. For example, the co-stimulatory signal domain may include an intracellular co-stimulatory signal domain from one or more proteins selected from the group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88. For example, the co-stimulatory signal region may be an intracellular co-stimulatory signal region from 2B4. For example, the co-stimulatory signal domain has the amino acid sequence as set forth in SEQ ID NO: 12.

It should be noted that, there is no particular limitation on the selection of the "linking peptide" in the present disclosure. For example, it may be a linking peptide in the form of (GGGGS)n, where n may range from 4 to 10. All forms of linking peptides known in the art that can be used to link functional amino acids without affecting the inherent functions of the functional amino acids are within the scope of protection of the present disclosure and may be used as the linking peptide in the present disclosure.

In the present disclosure, the intracellular signaling domain includes at least one ITAM motif, to transmit activation signals to an interior of cells. For example, the intracellular signaling domain may include an intracellular signaling domain from one or more proteins selected from the group consisting of: CD3ζ, CD3δ, CD3γ, CD3ε, CD79a, CD79b, FcεRIγ, FcεRIβ, FcγRIIa, DAP10, DAP-12, and other domains including at least one ITAM. For example, the intracellular signaling domain may be an intracellular signaling domain from CD3ζ. For example, the intracellular signaling domain has the amino acid sequence as set forth in SEQ ID NO: 13.

In the present disclosure, the CAR may further include a signal peptide at the N-terminus of the targeting moiety that binds to the GPRC5D protein. For example, the signal peptide may be a signal peptide from the CD8 protein. For example, the signal peptide may have the amino acid sequence as set forth in SEQ ID NO: 14.

According to a specific embodiment of the present disclosure, the chimeric antigen receptor has the amino acid sequence as set forth in SEQ ID NO: 15 or SEQ ID NO: 16. The transmembrane domain of the chimeric antigen receptor in the present disclosure may use the transmembrane domains as set forth in SEQ ID NO: 18 to SEQ ID NO: 23 according to different experimental requirements.

According to a specific embodiment of the present disclosure, the polypeptide encoding an anti-GPRC5D CAR includes: a) an extracellular antigen domain containing a GPRC5D binding moiety, the GPRC5D binding domain optionally including an anti-GPRC5D single-chain variable region fragment (scFV) or sdAb; b) a hinge region; c) a transmembrane domain; d) a co-stimulatory signal domain; and (d) an intracellular signaling domain.

In the polypeptide encoding an anti-GPRC5D CAR, the anti-GPRC5D scFV has the amino acid sequence as set forth in SEQ ID NO: 8.

According to a specific embodiment of the present disclosure, from the N-terminus to the C-terminus, the CAR may sequentially include a targeting moiety that binds to the GPRC5D protein (e.g., the GPRC5D scFV described in the present disclosure), the hinge region, the transmembrane domain, the co-stimulatory signal domain, and the intracellular signaling domain (as illustrated in FIG. 1A).

According to a more specific embodiment of the present disclosure, from the N-terminus to the C-terminus, the CAR may sequentially include the GPRCD scFV, a hinge region from CD8, a transmembrane domain from reversed NKG2D, a co-stimulatory signal domain from 2B4, and an intracellular signaling domain from CD3ζ.

In the present disclosure, from the N-terminus to the C-terminus, the CAR may sequentially include a targeting moiety that binds to the GPRC5D protein (e.g., the GPRC5D scFV described in the present disclosure) and a targeting moiety that binds to a second antigen (antigen 2) (e.g., scFV or sdAb against antigen 2), the hinge region, the transmembrane domain, the co-stimulatory signal domain, and the intracellular signaling domain, as illustrated in FIG. 1B. The scFV or sdAb against antigen 2 may be a scFV or sdAb that targets and binds to an antigen specifically expressed on lymphocytes.

For example, from the N-terminus to the C-terminus, the CAR may sequentially include the GPRC5D scFV and an scFV or sdAb capable of binding antigen 2, a hinge region from CD8, a transmembrane domain from reverse NKG2D, a co-stimulatory signal domain from 2B4, and an intracellular signaling domain from CD3ζ.

According to a more specific embodiment of the present disclosure, from the N-terminus to the C-terminus, the CAR may sequentially include a signal peptide, a GPRC5D scFV, a BCMA scFV or a BCMA sdAb, the hinge region, the transmembrane domain, the co-stimulatory signaling region, and the intracellular signaling region.

The BCMA sdAb has the amino acid sequence as set forth in SEQ ID NO: 9.

Patent CN 109153731 B provides an anti-BCMA sdAb sequence and a CAR molecule, which are assembled into T cells for the development of a CAR-T therapy targeting multiple myeloma. The present disclosure compares the performance of the anti-BCMA sdAb sequence and the anti-GPRC5D scFV sequence assembled with CAR on NK cells, and it is found that the anti-GPRC5D CAR NK cells obtained by the present disclosure exhibits superior killing activity against MM tumor cell lines compared with the anti-BCMA CAR NK cells. That is, the anti-GPRC5D CAR NK cells provided by the present disclosure exhibit better anti-tumor activity. Further, by introducing the anti-BCMA antibody sequence into the extracellular domain of the anti-GPRC5D CAR (anti-GPRCSD/BCMA CAR), it is found that the anti-tumor activity of the anti-GPRC5D CAR-iNK can be further enhanced.

### Polynucleotide, Expression Vector, Cell, and Composition

The present disclosure provides an isolated polynucleotide, which encodes the above-described chimeric antigen receptor.

The present disclosure provides an expression vector, which carries the above-described polynucleotide.

The present disclosure provides a cell, which carries the above-described polynucleotide or the above-described expression vector, or is capable of expressing the above-described chimeric antigen receptor. According to an embodiment of the present disclosure, the cell is capable of efficiently expressing the above-described chimeric antigen receptor under suitable conditions.

It should be noted that, the "suitable conditions" as used herein refer to conditions suitable for the expression of the chimeric antigen receptor described in the present disclosure. Those skilled in the art can readily understand that the conditions suitable for the expression of the chimeric antigen receptor include, but are not limited to, appropriate transformation or transfection methods, appropriate transformation or transfection conditions, healthy host cell states, appropriate host cell density, suitable cell culture environment, and suitable cell culture time. The "suitable conditions" are not particularly limited, and those skilled in the art can optimize the most suitable conditions for the expression of the chimeric antigen receptor according to the specific environment of the laboratory.

Nucleic acids encoding the antibody or antigen-binding fragment thereof, bispecific binding molecule described above, or chimeric antigen receptor described above can be delivered into cells using different tools, such as donor plasmids, piggyBac, dsDNA, ssDNA, and various integrating or non-integrating viruses. If necessary, according to the selected delivery tool, PBase can be selected to assist piggyBac delivery, and nucleases can be selected to form single-strand or double-strand breaks to assist other non-viral delivery methods.

The cell provided by the present disclosure may be a recombinant cell carrying the above-described polynucleotide or expression vector, or an engineered immune cell capable of expressing the above-described chimeric antigen receptor.

According to an embodiment of the present disclosure, a functionally enhanced immune cell can be obtained using any of the following methods or a combination thereof:
1) Randomly integrating one or more circular or linear exogenous DNA fragments into a target cell.
2) Site-specifically integrating one or more circular or linear exogenous DNA fragments into the target cell. The site-specific integration can be achieved by introducing a site-specific endonuclease to enhance the homologous recombination efficiency of exogenous DNA. Alternatively, the homologous recombination efficiency can also be enhanced by other physical means, such as electroporation or nucleofection.
3) Creating one or more double-strand breaks at specific sites in the target cell by introducing one or more endonucleases capable of recognizing the specific sites, such that the target cell generates base insertions or deletions at the specific sites through non-homologous end joining.
4) Creating one or more double-strand breaks at the specific sites in the target cell by introducing one or more endonucleases capable of recognizing the specific sites, and additionally providing exogenous circular or linear DNA fragments, such that the cell generates base insertions or deletions at the selected sites through homologous recombination.

In this case, the target cell may be the above-described immune cell, stem cell, or progenitor cell, or it may be a donor cell used for reprogramming into iPSCs, or it may also be any derivative cell generated during the differentiation of stem cells or progenitor cells into immune cells. The exogenous DNA fragment may include a gene expression cassette, i.e., at least elements such as a promoter and an open reading frame, or it may not contain a gene expression cassette.

According to an embodiment of the present disclosure, the promoter in the above-described gene expression cassette may be CAG, CMV, EF1α, PGK, UBC, or other constitutive, inducible, time, tissue-, or cell type-specific promoters, and the open reading frame in the above-described gene expression cassette includes one or more polypeptides or proteins to form a chimeric antigen receptor or to enhance immune cell function. The insertion site of the above-described exogenous DNA fragment may be the AAVS1 site or other sites where cell function is not affected by exogenous gene insertion, or it may be a site where the gene at the target site is disrupted after insertion, or it may also be the stop codon position of a functional gene.

CAR expression vectors can be constructed in various forms. As illustrated in FIG. 1 and FIG. 2, they may be inserted in-frame into the exon position of the target gene through a 2A or IRES sequence, and expression can be initiated by the promoter of the target gene (as illustrated in FIG. 1C). In some embodiments, the expression cassette may also include a promoter and a polyadenylation sequence (as illustrated in FIG. 1D). After integrated into the genome, the expression cassette initiates expression using its own carried expression elements. The expression cassette can also express a plurality of peptides simultaneously, and the coding sequences of two or more polypeptides can be linked by a 2A or IRES sequence. As an example, in an expression cassette including the promoter, anti-GPRC5D CAR-2A/IRES-hnCD16, and the polyadenylation sequence, the anti-GPRC5D CAR and hnCD16 can be expressed simultaneously under the drive of an exogenous promoter (as illustrated in FIG. 1E).

In some embodiments, the genetically modified immune cell or a source cell thereof generated by the above-described methods expresses a genetically engineered antigen receptor. This genetically engineered antigen receptor may be a chimeric antigen receptor (CAR), a T-cell receptor (TCR), or a NK-cell receptor (NCR).

In some embodiments, for the genetically modified immune cell or its source cell generated by the method provided herein, the immune cell is an NK cell, and its source cell is a pluripotent stem cell or a derivative thereof. Specifically, the pluripotent stem cell is an iPSC, a cloned iPSC, or an iPSC cell line. The derivative cell of the pluripotent stem cell includes a CD34⁺ cell, a hemogenic endothelial cell, a hematopoietic stem and progenitor cell (HSPC), a hematopoietic pluripotent progenitor cell, an NK progenitor cell, and an NK cell. Its source cell may also be an NK cell, an NK progenitor cell, a hematopoietic pluripotent progenitor cell, a CD34⁺ cell, a hemogenic endothelial cell, a hematopoietic stem and progenitor cell isolated from peripheral blood, umbilical cord blood, or other tissues (including tumor tissues) or organs.

In some embodiments, the genetically modified immune cell or its source cell generated by the method provided herein is used. The immune cell is a T cell, including CD4⁺ T cell, CD8⁺ T cell, CD3⁺CD4⁻CD8⁻ T cell, or CD3⁺CD56⁺ T cell. A preferred cell may be selected from a naive T cell (TN cell), a T memory stem cell (TSCM cell), a memory T cell (TCM cell), a regulatory T cell (Treg cell), a TCR-T cell, a tumor-infiltrating lymphocyte (TIL), or an effector memory T cell (TEM cells), and a combination thereof. Its source cell is a pluripotent stem cell or a hematopoietic stem cell derived therefrom. Specifically, the pluripotent stem cell is an iPSC, a clonal iPSC, or an iPSC cell line. The derivative of pluripotent stem cell includes a CD34⁺ cell, a hemogenic endothelial cell, a hematopoietic stem and progenitor cell (HSPC), a hematopoietic pluripotent progenitor cell, a T progenitor cell, a T cell, and an NKT cell. Its source cell may also be a T cell, a T progenitor cell, a hematopoietic multipotent progenitor cell, a CD34⁺ cell, a hemogenic endothelial cell, and a hematopoietic stem and progenitor cell isolated from peripheral blood, umbilical cord blood, or other tissues (including tumor tissues) or organs.

In some embodiments, the genetically modified immune cell or its source cell generated by the method provided herein is used. The immune cell is a macrophage, including M1 macrophage and M2 macrophage. Its source cell includes macrophages from various tissues, such as those from bone marrow, lymph nodes, liver, spleen, lung, and circulatory system, or microglia. It also includes a macrophage from a pluripotent stem cell or a hematopoietic stem cell derived therefrom. Specifically, the pluripotent stem cell is an iPSC, a clonal iPSC, or an iPSC cell line. The derivative of the pluripotent stem cell includes a CD34⁺ cell, a hemogenic endothelial cell, a hematopoietic stem and progenitor cell (HSPC), a hematopoietic pluripotent progenitor cell, a macrophage progenitor cell, and a macrophage. Its source cell may also be a macrophage, a macrophage progenitor cell, a hematopoietic pluripotent progenitor cell, a CD34⁺ cell, a hemogenic endothelial cell, a hematopoietic stem and progenitor cell isolated from peripheral blood, umbilical cord blood, or other tissues (including tumor tissues) or organs.

In some embodiments, the immune cell of the present disclosure is an embryonic stem cell, an induced pluripotent cell (iPSC), a cloned iPSC, an iPSC cell line, or a derivative cell differentiated from any of the above-described embryonic stem cell or iPSC.

Any of the above-described cells expresses at least one polypeptide capable of recognizing a chimeric antigen receptor of GPRC5D (an anti-GPRC5D CAR).

In some embodiments where the derivative cell is differentiated from the iPSC, the derivative cell exhibits an enhanced killing activity against the tumor cell expressing GPRC5D compared with the corresponding primary cell obtained from peripheral blood, umbilical cord blood, or any other tissues or organs.

In some embodiments where the iPSC and the derivative cell thereof include at least a chimeric antigen receptor capable of recognizing GPRCSD, the cell may further include one or more of the following genome edits: (i) B2M and CIITA knockout or low-expression phenotypes; (ii) introduction of expression of CD47, HLA-E, HLA-G (including non-cleavable forms), or any combination thereof; (iii) introduction of expression of high-affinity non-cleavable CD16 (hnCD16) or a variant thereof; (iv) introduction of expression of a second or third CAR; (v) introduction of expression of a second or third scFV or single-domain antibody (sdAb); (vi) knockout or reduction of expression of TGFBR2, PVR, NKG2A, TIM3, TIGIT, CD38, or TCR; and (vii) introduction or increase of expression of at least one of 4-1BBL, NKG2D, NKp44, NKp46, CD3, CD47, CD113, CD131, CD137, CD80, CD86, CD68, CD226, PDL1, A2AR, CAR, TCR, Fc receptor, a conjugate, and a surface trigger receptor used to couple with a universal conjugate.

In some embodiments of the cell or cell population thereof, one or more cells including anti-GPRC5D CAR are primary NK cells, or NK cells or cell populations thereof differentiated from a pluripotent stem cell, a hematopoietic stem and progenitor cell, or an NK progenitor cell. Compared with non-genetically modified counterparts, these cells at least exhibit an enhanced killing activity against GPRC5D-expressing tumor cells.

The present disclosure provides a composition for therapeutic use. The composition includes an immune cell, preferably an NK cell, and an additional pharmaceutically acceptable excipient. In some embodiments, a subject suitable for receiving cell therapy suffers from at least one of the following conditions: multiple myeloma, and/or the presence of a tumor cell expressing GPRC5D in their body. The composition may further include a clinically acceptable peptide, a cytokine, a growth factor, a feeder cell or an alternative factor thereof, an immune checkpoint inhibitor, or an antibody drug (such as an anti-CD38 antibody). In some embodiments of the composition for therapeutic use, the antibody used with the provided cell may include daratumumab, and/or at least one selected from the group consisting of: rituximab, vetuzumab, ofatumumab, ublituximab, ocaratuzumab, obinutuzumab, trastuzumab, pertuzumab, alemtuzumab, cetuximab, dinutuximab, avelumab, daratumumab, isatuximab, and epratuzumab.

The present disclosure further provides a method for obtaining or manufacturing the cell described above. This method includes directed differentiation of one or more iPSCs containing a chimeric antigen receptor capable of recognizing GPRC5D.

The present disclosure further provides a method for obtaining one or more iPSCs containing an anti-GPRC5D CAR. This method includes introducing a DNA sequence encoding an anti-GPRC5D CAR polypeptide into the iPSC using any of the following gene editing methods or a combination thereof: 1) inserting an exogenous DNA fragment into a target site using a homologous recombination method, in which targeting of the target site can be achieved by flanking homologous sequences at two sides of the exogenous DNA fragment; 2) inserting an exogenous DNA fragment into a site of a double-strand break in the genome using a non-homologous recombination method; and 3) randomly integrating an exogenous DNA fragment into a host genome using tools such as viruses, transposons, or plasmids.

The present disclosure further provides a method for obtaining one or more iPSCs containing a chimeric antigen receptor polypeptide capable of recognizing GPRC5D. This method includes further performing at least one of the following additional genetic modifications, or a combination thereof: 1) introducing high-affinity non-cleavable CD16 or a variant thereof; 2) knocking out CD38; 3) knocking out B2M and CIITA, and/or PVR; and introducing CD47, and/or HLA-G or non-cleavable HLA-G, and/or HLA-E; and 4) introducing membrane-bound IL15 (mbIL15). The above selected genetic modifications may be performed in one step or in separate sequential steps. The introduced exogenous gene expression cassette or exogenous polypeptide can be expressed independently or co-expressed in a bicistronic construct. The expression may be driven by an exogenous promoter, or stably proceed by a promoter of an endogenous gene or proceed tissue-specifically. The exogenous promoter may be any one of the following promoters or a combination thereof: CAG, CMV, EF1α, PGK, UBC, or other constitutive, inducible, time-, tissue-, or cell type-specific promoters. In some embodiments, targeted genetic modification may be performed using an engineered endonuclease (such as a clustered regularly interspaced short palindromic repeat (CRISPR) system, a zinc finger nuclease (ZFN), a transcription activator-like effector nuclease (TALEN), and a meganuclease), homologous recombination, or any other functional variations of these methods.

The present disclosure further provides a piggyBac-mediated method for expressing an anti-GPRC5D CAR to obtain one or more iPSCs containing the above-described anti-GPRC5D CAR polypeptide. In some embodiments of the piggyBac-mediated method, the DNA sequence encoding the anti-GPRC5D CAR is located in the transposable region of piggyBac (the region between the inverted terminal repeat sequences at two ends). Subsequently, with the assistance of a transposase, piggybac randomly integrates the DNA sequence encoding the anti-GPRC5D CAR into the TTAA site of the host genome.

FIG. 2A illustrates several expression cassettes containing an anti-GPRC5D CAR peptide. These cassettes can be cloned into a piggyBac plasmid and randomly integrated into a host genome with the aid of PBase. FIG. 2B shows an example of CAG-anti-GPRCSD CAR-BGHpA inserted into a piggyBac transposon region to obtain an anti-GPRC5D CAR plasmid.

DNA encoding the anti-GPRC5D CAR can be introduced using various tools, such as donor plasmids, piggyBac, dsDNA, ssDNA, and various integrating or non-integrating viruses. Depending on the selected delivery tool, PBase can be selected to assist piggyBac delivery, and nucleases can be selected to form single-strand or double-strand breaks to assist other non-viral delivery methods.

FIG. 3A shows a schematic diagram of a homologous recombination template containing an anti-GPRC5D CAR. FIG. 3B provides several examples of DNA templates containing an anti-GPRC5D CAR expression cassette for homologous recombination. These DNA templates may be in the form of single-stranded DNA, double-stranded DNA, or plasmids. FIG. 3C provides an example of a DNA template with an anti-GPRC5D CAR and hnCD16 expression cassette, which targets any site in the human genome, and the location of the expression cassette in the genome after homologous recombination.

All forms of the constructs illustrated in FIG. 1 to FIG. 3 of the present disclosure are all within the scope of protection of the constructs protected by the present disclosure.

The present disclosure further provides a CRISPR-mediated gene editing method for obtaining one or more immune cells or iPSC constructs containing a chimeric antigen receptor polypeptide capable of recognizing GPRC5D. In some embodiments of the CRISPR-mediated method, CRISPR binds to the target sequence and generates a double-strand break, followed by the insertion of the DNA sequence encoding the anti-GPRC5D CAR at the target sequence via non-homologous end joining or homologous recombination. The target sequence may be any of the following sites: AAVS1 site, or any of the following sites: CISH, SOCS1, SOCS2, SOCS4, SOCSS, SOCS6, SOCS7, NKG2A, B2M, CIITA, CD38, TGFBR2, PVR, TIM3, TIGIT, and TCR.

In some embodiments of the CRISPR-mediated method, the editing further includes expressing any of the following genes at the same site as described above, either in tandem or in parallel with the anti-GPRC5D CAR polypeptide: hnCD16 or a variant thereof, mbIL15, CD47, HLA-E, HLA-G or non-cleavable HLA-G, tEGFR, or an induced suicide gene (iCaspase9).

In some embodiments of the CRISPR-mediated method, the editing further includes expressing the gene or polypeptide selected from at least one of hnCD 16 or a variant thereof, mbIL15, CD47, HLA-E, HLA-G or non-cleavable HLA-G, tEGFR, or iCas9 at a different site including at least one of AAVS1, CISH, SOCS1, SOCS2, SOCS4, SOCSS, SOCS6, SOCS7, NKG2A, B2M, CIITA, CD38, TGFBR2, PVR, TIM3, TIGIT, and TCR.

Further, the present disclosure provides a donor plasmid or donor DNA fragment for engineering a cell to obtain a genome-edited cell. The donor plasmid or donor DNA fragment includes: 1) one or more nucleic acid sequences encoding one or more exogenous polypeptides, and 2) left and right homologous arm sequences targeting a target site. The genome-edited cell is an induced pluripotent cell (iPSC), a cloned iPSC, or an iPSC cell line. The genome-edited cell includes targeted integration of one or more nucleic acid sequences encoding one or more exogenous polypeptides at the target site. The genome-edited cell is an iPSC capable of differentiating into a CD34⁺ cell. In some embodiments, the one or more nucleic acid sequences encoding one or more exogenous proteins are in-frame linked to and driven by an endogenous promoter at a target locus after integration; or the selected position at the target locus is located in an exon of the target gene. In some embodiments, the one or more nucleic acid sequences encoding one or more exogenous polypeptides are driven by an exogenous promoter after integration, and the integration position at the target locus is in an exon of the target gene.

In some embodiments, the present disclosure provides a piggybac plasmid for engineering a cell to obtain a genome-edited cell. The piggybac plasmid includes: one or more nucleic acid sequences encoding one or more exogenous polypeptides. The exogenous polypeptides include an anti-GPRC5D CAR, and/or at least one of hnCD16 or a variant thereof, CD47, HLA-E, HLA-G or non-cleavable HLA-G, tEGFR, iCas9, and mbIL15. The genome-edited cell is the induced pluripotent cell (iPSC), cloned iPSC, or iPSC cell line. The genome-edited cell includes random integration of one or more nucleic acid sequences encoding one or more exogenous polypeptides into a host cell genome. The genome-edited cell is the iPSC capable of differentiating into the CD34⁺ cell.

In some embodiments using the donor plasmid, donor DNA fragment, or piggyBac, the gene-edited iPSC-derived blood cell includes a derived CD34⁺ cell, a derived hematopoietic stem and progenitor cell, a derived hematopoietic pluripotent progenitor cell, a derived T progenitor cell, a derived NK progenitor cell, a derived T cell, a derived NKT cell, a derived NK cell, or a derived B cell. In an embodiment, the hematopoietic cell derived from the differentiated genome-edited iPSC includes the derived NK cell or derived T cell and exhibits at least one of the following features compared with their native counterpart cells obtained from peripheral blood, umbilical cord blood, or any other donor tissues: (1) the specific killing activity against the tumor cell expressing GPRC5D; and (2) obtaining or enhancement of the ADCC effect.

The present disclosure further provides a composition for manufacturing a gene-edited iPSC or a hematopoietic cell derived therefrom. The composition includes the donor plasmid, donor DNA, or piggyBac plasmid as described herein. Further, the present disclosure provides a kit including the donor plasmid, or donor DNA fragment, or piggyBac plasmid described herein, and one or more culture media for culture, maintenance, or differentiation of iPSC. The present disclosure further provides a donor plasmid, donor DNA, piggyBac plasmid, or a host cell for use as a medicament.

The present disclosure provides a method for generating a genome-edited iPSC or a hematopoietic cell derived therefrom using the donor plasmid, donor DNA fragment, or piggyBac plasmid as described herein. The method includes: 1) introducing the donor plasmid, donor DNA fragment, or piggyBac plasmid into an iPSC, and 2) culturing the iPSC for a sufficient period of time to obtain the genome-edited iPSC. In some embodiments, the method may further include guiding the resulting genome-edited iPSC to differentiate into the hematopoietic cell having the same genotype as the genome-edited iPSC. In some embodiments of the method, the method further includes introducing a CRISPR system, ZFN, TALEN, homing nuclease, or any other functional variant thereof.

The anti-tumor efficacy of effector immune cells (such as NK cells, T cells, and macrophages) in adoptive cell therapy remains relatively limited both in vitro and in vivo. Engineered methods are needed to enhance cell function, thereby benefiting tumor patients. One objective of the present disclosure is to prepare engineered immune cells with GPRC5D antibody or bispecific binding molecule containing the GPRC5D antibody as the extracellular domain. Such immune cells can be isolated from peripheral blood, umbilical cord blood, or specific tissues or organs (including tumor tissues or other organs) of patients or donors, or differentiated from pluripotent stem cells (such as induced pluripotent stem cells (iPSCs) or embryonic stem cells (embryonic stem cells within 14 days after fertilization and not subjected to in vivo development), hematopoietic stem cells, or various types of immune cell progenitors. The engineering is primarily achieved through genetic modification. The engineering can be completed at the stem cell stage or progenitor cell stage, or at the immune cell stage. The engineering includes one or more genetic modifications, including DNA insertion, deletion, and substitution. These genetic modifications can be completed in one step or through multiple steps in different orders. The genetic modifications completed at the stem cell stage or progenitor cell stage remain and maintain the corresponding functions in the derivative cells produced during subsequent differentiation, expansion, passage, or transplantation processes.

### Kit for Detecting GPRC5D

According to a specific embodiment of the present disclosure, the present disclosure provides a kit for detecting GPRC5D. The kit includes the above-described antibody or antigen-binding fragment thereof and/or the above-described chimeric antigen receptor. The antibody or antigen-binding fragment thereof capable of specifically recognizing GPRC5D or the bispecific binding molecule containing a GPRC5D antibody is capable of binding to GPRC5D in a sample to be tested, thereby achieving the objective of detecting GPRC5D levels in vitro.

### Use and Method

According to a specific embodiment of the present disclosure, the present disclosure provides use of the above-described chimeric antigen receptor, polynucleotide, expression vector, cell, and/or composition in the treatment of a hematologic malignancy or solid tumor, an autoimmune disease, or a viral infectious disease in a subject. According to an embodiment of the present disclosure, by means of the antibody or antigen-binding fragment thereof capable of specifically recognizing GPRC5D or the bispecific binding molecule containing the GPRC5D antibody, the chimeric antigen receptor is capable of binding to GPRC5D and a second antibody in the bispecific binding molecule, thereby achieving effective treatment of the hematologic malignancy or solid tumor, autoimmune disease, or viral infection.

According to an embodiment of the present disclosure, the hematologic malignancy or solid tumor is a GPRC5D-associated disease. The chimeric antigen receptor can effectively treat GPRC5D-mediated associated diseases.

According to a specific embodiment of the present disclosure, the GPRC5D-associated disease includes, but is not limited to, multiple myeloma. According to a specific embodiment of the present disclosure, the chimeric antigen receptor can effectively treat multiple myeloma.

According to an embodiment of the present disclosure, the autoimmune disease includes, but is not limited to, at least one of: systemic lupus erythematosus, rheumatoid arthritis, systemic sclerosis, arthritis syndrome, autoimmune anemia, rheumatoid heart disease, Sjögren's syndrome, systemic vasculitis, pemphigus, dermatomyositis, autoimmune hemolytic anemia, autoimmune thyroid disease, Hashimoto's thyroiditis, type 1 diabetes, and multiple sclerosis.

According to an embodiment of the present disclosure, the viral infectious disease includes, but is not limited to, an EBV infectious disease.

According to a specific embodiment of the present disclosure, the present disclosure provides a method for preventing and/or treating a hematologic malignancy or solid tumor, an autoimmune disease, or a viral infectious disease in a subject. The method includes administering to the subject at least one of the above-described chimeric antigen receptor, polynucleotide, expression vector, cell, and composition. As previously mentioned, by means of the antibody or antigen-binding fragment thereof capable of specifically recognizing GPRC5D or the bispecific binding molecule containing the GPRC5D antibody, the chimeric antigen receptor is capable of binding to GPRC5D and the second antibody in the bispecific binding molecule, thereby achieving the effective treatment of the hematologic malignancy or solid tumor, autoimmune disease, or viral infection. Therefore, the efficacy of the above-described chimeric antigen receptor, polynucleotide, expression vector, cell, and composition in preventing and/or treating the hematologic malignancy or solid tumor, autoimmune disease, or viral infection will be omitted here.

According to an embodiment of the present disclosure, the hematologic malignancy or solid tumor is a GPRC5D-associated disease.

According to an embodiment of the present disclosure, the autoimmune disease includes, but is not limited to, at least one of: systemic lupus erythematosus, rheumatoid arthritis, systemic sclerosis, arthritis syndrome, autoimmune anemia, rheumatoid heart disease, Sjögren's syndrome, systemic vasculitis, pemphigus, dermatomyositis, autoimmune hemolytic anemia, autoimmune thyroid disease, Hashimoto's thyroiditis, type 1 diabetes, and multiple sclerosis.

According to an embodiment of the present disclosure, the viral infectious disease includes, but is not limited to, an EBV infectious disease.

According to a specific embodiment of the present disclosure, the GPRC5D-associated disease includes multiple myeloma.

According to a specific embodiment of the present disclosure, the present disclosure provides use of the above-described chimeric antigen receptor, polynucleotide, or expression vector in the detection of GPRC5D or in the diagnosis of whether a subject is suffering from a GPRC5D-associated disease. As previously described, the chimeric antigen receptor according to an embodiment of the present disclosure can effectively bind to GPRC5D. Therefore, the chimeric antigen receptor can be used to detect GPRC5D. Since GPRC5D mediates a variety of diseases, it can be determined that whether the subject suffers from a GPRC5D-mediated disease based on the level of GPRC5D contained in a biological sample from the subject.

According to a specific embodiment of the present disclosure, the GPRC5D-associated disease includes multiple myeloma.

According to a specific embodiment of the present disclosure, the present disclosure provides a method for detecting GPRC5D. The method includes detecting GPRC5D in a sample to be tested using at least one of the above-described chimeric antigen receptor, polynucleotide, and expression vector. As previously described, the chimeric antigen receptor according to an embodiment of the present disclosure can effectively bind to GPRC5D. Therefore, the chimeric antigen receptor can be used to detect GPRC5D.

The method for detecting GPRC5D according to an embodiment of the present disclosure can be used for scientific research. The sample includes, but is not limited to, tissues, cells, blood, serum, plasma, urine, feces, saliva, or sweat.

According to a specific embodiment of the present disclosure, the present disclosure provides a method for diagnosing whether a subject is suffering from a GPRC5D-associated disease. The method includes detecting GPRC5D in a sample collected from the subject using at least one of the above-described chimeric antigen receptor, polynucleotide, and expression vector. As previously described, the chimeric antigen receptor according to an embodiment of the present disclosure can effectively bind to GPRC5D. Therefore, the chimeric antigen receptor can be used to detect GPRC5D. Since GPRC5D mediates a plurality of diseases, it can be determined that whether the subject is suffering from a GPRC5D-mediated disease based on the level of GPRC5D contained in a biological sample from the subject. Further, the content of GPRC5D in the sample to be tested from the subject can be monitored using the above-described substances, meaning the above-described substances can also be used for disease staging in the subject suffering from the GPRC5D-mediated associated disease, and can also be used for prognostic evaluation of the GPRC5D-mediated associated disease.

According to a specific embodiment of the present disclosure, the GPRC5D-associated disease includes multiple myeloma. According to specific embodiments of the present disclosure, the above-described method can effectively diagnose, stage, or evaluate the prognosis of GPRC5D-associated disease in the subject.

According to specific embodiments of the present disclosure, the sample includes, but is not limited to, tissue, cells, blood, serum, plasma, urine, feces, saliva, or sweat.

The solutions of the present disclosure will be explained with examples. Those skilled in the art can understand that the following examples are only used to illustrate the present disclosure and should not be regarded as limitations of the scope of the present disclosure. If the specific technologies or conditions are not specified in the examples, they shall be carried out according to the technologies or conditions as described in the literature in the art or according to the product instructions. The reagents and instruments used without indicating the manufacturer are all conventional products that can be purchased commercially.

### Example 1: Structure of Anti-GPRC5D CAR Polypeptides

An anti-GPRC5D antibody that specifically bound to GPRC5D was obtained through hybridoma cell screening technology. Based on the amino acid sequence of the anti-GPRC5D antibody, the inventors obtained two anti-GPRC5D CAR polypeptides, as shown in (1) and (2) below.
(1) FIG. 1A illustrates the structure of an anti-GPRC5D CAR polypeptide including, from the N-terminus to the C-terminus, an antigen recognition domain (anti-GPRC5D scFV), a hinge region, a transmembrane domain, a co-stimulatory signal domain, and an intracellular signaling domain.

The amino acid sequence of the anti-GPRC5D CAR polypeptide is as set forth in SEQ ID NO: 15:

MALPVTALLLPLALLLHAARPDIQMTQSPSSLSASVGDRVTITCKASQNVG
TNVAWYQQKPGQSPKALIYSASYRDSGVPDRFSGSGSGTDFTLTISSLQPEDFATYYCQ
QYKSYPLTFGQGTRLEIKGGGGSGGGGSGGGGSQVQLQESGPGLVKPSETLSLTCTVS
GYSITSDYAWNWIRQPPGKGLEWMGYISYSGSATYSPSLKSRITISRDTSKNQFSLKLS
SVTAADTAVYYCARGGIAGRGRWGAMDYWGQGTTVTVSSTTTPAPRPPTPAPTIASQ
PLSLRPEACRPAAGGAVHTRGLDFACDSNLFVASWIAVMIIFRIGMAVAIFCCFFLPSWR
RKRKEKQSETSPKEFLTIYEDVKDLKTRRNHEQEQTFPGGGSTIYSMIQSQSSAPTSQE
PAYTLYSLIQPSRKSGSRKRNHSPSFNSTIYEVIGKSQPKAQNPARLSRKELENFDVYSR
VKFSRSADAPAYKQGQNQLYNELNLGRREEYDVLDKRRGRDPEMGGKPRRKNPQEG
LYNELQKDKMAEAYSEIGMKGERRRGKGHDGLYQGLSTATKDTYDALHMQALPPR
(SEQ ID NO: 15).

The transmembrane domain in the present disclosure is underlined. In the present disclosure, depending on different experimental requirements, the transmembrane domain of the chimeric antigen receptor may be the transmembrane domain as set forth in any one of SEQ ID NO: 18 to SEQ ID NO: 23.

For example, the amino acid sequence of another anti-GPRC5D CAR is as set forth in SEQ ID NO: 25 (the amino acid sequence of the transmembrane domain is as set forth in SEQ ID NO: 18):

MALPVTALLLPLALLLHAARPDIQMTQSPSSLSASVGDRVTITCKASQNVG
TNVAWYQQKPGQSPKALIYSASYRDSGVPDRFSGSGSGTDFTLTISSLQPEDFATYYCQ
QYKSYPLTFGQGTRLEIKGGGGSGGGGSGGGGSQVQLQESGPGLVKPSETLSLTCTVS
GYSITSDYAWNWIRQPPGKGLEWMGYISYSGSATYSPSLKSRITISRDTSKNQFSLKLS
SVTAADTAVYYCARGGIAGRGRWGAMDYWGQGTTVTVSSTTTPAPRPPTPAPTIASQ
PLSLRPEACRPAAGGAVHTRGLDFACDSNLFVASWIAVMIIFRIGMAVAIFCCFFFPSWR
RKRKEKQSETSPKEFLTIYEDVKDLKTRRNHEQEQTFPGGGSTIYSMIQSQSSAPTSQE
PAYTLYSLIQPSRKSGSRKRNHSPSFNSTIYEVIGKSQPKAQNPARLSRKELENFDVYSR
VKFSRSADAPAYKQGQNQLYNELNLGRREEYDVLDKRRGRDPEMGGKPRRKNPQEG
LYNELQKDKMAEAYSEIGMKGERRRGKGHDGLYQGLSTATKDTYDALHMQALPPR
(SEQ ID NO: 25).

(2) FIG. 1B illustrates the structure of an anti-GPRC5D CAR polypeptide including, from the N-terminus to the C-terminus, an antigen recognition domain (anti-GPRC5D scFV), a BCMA sdAb, a hinge region, a transmembrane domain, a co-stimulatory signal domain, and an intracellular signaling domain.

The amino acid sequence of the GPRC5D scFV-BCMA sdAb CAR is as set forth in SEQ ID NO: 16, in which the transmembrane domain is underlined:

MALPVTALLLPLALLLHAARPDIQMTQSPSSLSASVGDRVTITCKASQNVG
TNVAWYQQKPGQSPKALIYSASYRDSGVPDRFSGSGSGTDFTLTISSLQPEDFATYYCQ
QYKSYPLTFGQGTRLEIKGGGGSGGGGSGGGGSQVQLQESGPGLVKPSETLSLTCTVS
GYSITSDYAWNWIRQPPGKGLEWMGYISYSGSATYSPSLKSRITISRDTSKNQFSLKLS
SVTAADTAVYYCARGGIAGRGRWGAMDYWGQGTTVTVSSGGGGSGGGGSGGGGS
GGGGSGGGGSQVKLEESGGGLVQAGRSLRLSCAASEHTFSSHVMGWFRQAPGKERE
SVAVIGWRDISTSYADSVKGRFTISRDNAKKTLYLQMNSLKPEDTAVYYCAARRIDAA

DFDSWGQGTQVTVSSTTTPAPRPPTPAPTIASQPLSLRPEACRPAAGGAVHTRGLDFAC
DSNLFVASWIAVMIIFRIGMAVAIFCCFFLPSWRRKRKEKQSETSPKEFLTIYEDVKDLK
TRRNHEQEQTFPGGGSTIYSMIQSQSSAPTSQEPAYTLYSLIQPSRKSGSRKRNHSPSFN
STIYEVIGKSQPKAQNPARLSRKELENFDVYSRVKFSRSADAPAYKQGQNQLYNELNL
GRREEYDVLDKRRGRDPEMGGKPRRKNPQEGLYNELQKDKMAEAYSEIGMKGERR
RGKGHDGLYQGLSTATKDTYDALHMQALPPR (SEQ ID NO: 16).

In the present disclosure, depending on different experimental requirements, the transmembrane domain of the GPRC5D scFv-BCMA sdAb CAR may be the transmembrane domain as set forth in any one of SEQ ID NO: 18 to SEQ ID NO: 23 and SEQ ID NO: 27 to SEQ ID NO: 35. For example, the amino acid sequence of another GPRC5D scFv-BCMA sdAb CAR is as set forth in SEQ ID NO: 26 (the amino acid sequence of the transmembrane domain is as set forth in SEQ ID NO: 18):

MALPVTALLLPLALLLHAARPDIQMTQSPSSLSASVGDRVTITCKASQNVG
TNVAWYQQKPGQSPKALIYSASYRDSGVPDRFSGSGSGTDFTLTISSLQPEDFATYYCQ
QYKSYPLTFGQGTRLEIKGGGGSGGGGSGGGGSQVQLQESGPGLVKPSETLSLTCTVS
GYSITSDYAWNWIRQPPGKGLEWMGYISYSGSATYSPSLKSRITISRDTSKNQFSLKLS
SVTAADTAVYYCARGGIAGRGRWGAMDYWGQGTTVTVSSGGGGSGGGGSGGGGS
GGGGSGGGGSQVKLEESGGGLVQAGRSLRLSCAASEHTFSSHVMGWFRQAPGKERE
SVAVIGWRDISTSYADSVKGRFTISRDNAKKTLYLQMNSLKPEDTAVYYCAARRIDAA
DFDSWGQGTQVTVSSTTTPAPRPPTPAPTIASQPLSLRPEACRPAAGGAVHTRGLDFAC
DSNLFVASWIAVMIIFRIGMAVAIFCCFFFPSWRRKRKEKQSETSPKEFLTIYEDVKDLK
TRRNHEQEQTFPGGGSTIYSMIQSQSSAPTSQEPAYTLYSLIQPSRKSGSRKRNHSPSFN
STIYEVIGKSQPKAQNPARLSRKELENFDVYSRVKFSRSADAPAYKQGQNQLYNELNL
GRREEYDVLDKRRGRDPEMGGKPRRKNPQEGLYNELQKDKMAEAYSEIGMKGERR
RGKGHDGLYQGLSTATKDTYDALHMQALPPR (SEQ ID NO: 26).

### Example 2: Introduction of Anti-GPRC5D CAR Molecules

The expression cassette illustrated in FIG. 2A (a) was cloned into the piggyBac vector carrying puromycin resistance to obtain the anti-GPRC5D CAR plasmid (illustrated in FIG. 2B). In the present disclosure, the transmembrane domain sequence in the CAR molecule may have multiple variants. As an example, the transmembrane domain sequence as set forth in SEQ ID NO: 18 was used. The resulting plasmid sequence is set forth in SEQ ID NO: 17. Subsequently, the anti-GPRC5D CAR plasmid and the plasmid expressing transposase (PBase) were co-transfected into healthy human-derived iPSCs (WT iPSCs, from Cell Inspire Biotechnology) using a nucleofector (Amaxa, Nucleofector^{®} II) with a nucleofection kit (LONZA, CAT#VPH5022). For the transfection procedure, reference may be made to the kit's instructions for use. After selection with 0.5 µg/ml puromycin for 2 days to 7 days, the cells were expanded and cultured to obtain iPSCs expressing the anti-GPRC5D CAR molecule (anti-GPRC5D CAR-iPSC). Using a mouse anti-GPRC5D scFV antibody (primary antibody, Kangyuan Bochuang) and PE Goat anti-mouse IgG (secondary antibody, Biolegend), the proportion of iPSCs expressing the anti-GPRC5D CAR molecule was determined using FACS (illustrated in FIG. 5A), indicating that the iPSCs expressing the anti-GPRC5D CAR molecule were obtained. Using the same method, iPSCs expressing the CAR molecule against anti-GPRC5D and BCMA antigens (anti-GPRC5D/BCMA CAR-iPSCs) were obtained.

GGCTTCTGAGGCGGTTAATTAAAATAAAACTAGAGCGGGCCCGGTACTA
ACCAAACTGGATCTCTGCTGTCCCTGTAATAAACCCGAAAATTTTGAATTTTTGTAA
TTTGTTTTTGTAATTCTTTAGTTTGTATGTCTGTTGCTATTATGTCTACTATTCTTTCC
CCTGCACTGTACCCCCCAATCCCCCCTTTTCTTTTAAAATTGTGGATGAATACTGCC
ATTTGTCTGCAGAGCGGCCGCGTCGAGATAACTTCGTATAATGTATGCTATACGAAG
TTATGTTTTGTTACTTTATAGAAGAAATTTTGAGTTTTTGTTTTTTTTTAATAAATAAA
TAAACATAAATAAATTGTTTGTTGAATTTATTATTAGTATGTAAGTGTAAATATAATAA
AACTTAATATCTATTCAAATTAATAAATAAACCTCGATATACAGACCGATAAAACACA
TGCGTCAATTTTACGCATGATTATCTTTAACGTACGTCACAATATGATTATCTTTCTA
GGGTTAAAGAGCTCCAGCTTTTGTTCCCTTTAGTGAGGGTTAATTTCGAGCTTGGC
GTAATCATGGTCATAGCTGTTTCCTGTGTGAAATTGTTATCCGCTCACAATTCCACA
CAACATACGAGCCGGAAGCATAAAGTGTAAAGCCTGGGGTGCCTAATGAGTGAGC
TAACTCACATTAATTGCGTTGCGCTCACTGCCCGCTTTCCAGTCGGGAAACCTGTC
GTGCCAGCTGCATTAATGAATCGGCCAACGCGCGGGGAGAGGCGGTTTGCGTATT
GGGCGCTCTTCCGCTTCCTCGCTCACTGACTCGCTGCGCTCGGTCGTTCGGCTGCG
GCGAGCGGTATCAGCTCACTCAAAGGCGGTAATACGGTTATCCACAGAATCAGGG

GATAACGCAGGAAAGAACATGTGAGCAAAAGGCCAGCAAAAGGCCAGGAACCGT
AAAAAGGCCGCGTTGCTGGCGTTTTTCCATAGGCTCCGCCCCCCTGACGAGCATCA
CAAAAATCGACGCTCAAGTCAGAGGTGGCGAAACCCGACAGGACTATAAAGATAC
CAGGCGTTTCCCCCTGGAAGCTCCCTCGTGCGCTCTCCTGTTCCGACCCTGCCGCT
TACCGGATACCTGTCCGCCTTTCTCCCTTCGGGAAGCGTGGCGCTTTCTCATAGCTC
ACGCTGTAGGTATCTCAGTTCGGTGTAGGTCGTTCGCTCCAAGCTGGGCTGTGTGC
ACGAACCCCCCGTTCAGCCCGACCGCTGCGCCTTATCCGGTAACTATCGTCTTGAG
TCCAACCCGGTAAGACACGACTTATCGCCACTGGCAGCAGCCACTGGTAACAGGA
TTAGCAGAGCGAGGTATGTAGGCGGTGCTACAGAGTTCTTGAAGTGGTGGCCTAA
CTACGGCTACACTAGAAGAACAGTATTTGGTATCTGCGCTCTGCTGAAGCCAGTTA
CCTTCGGAAAAAGAGTTGGTAGCTCTTGATCCGGCAAACAAACCACCGCTGGTAG
CGGTGGTTTTTTTGTTTGCAAGCAGCAGATTACGCGCAGAAAAAAAGGATCTCAA
GAAGATCCTTTGATCTTTTCTACGGGGTCTGACGCTCAGTGGAACGAAAACTCACG
TTAAGGGATTTTGGTCATGAGATTATCAAAAAGGATCTTCACCTAGATCCTTTTAAA
TTAAAAATGAAGTTTTAAATCAATCTAAAGTATATATGAGTAAACTTGGTCTGACAG
TTACCAATGCTTAATCAGTGAGGCACCTATCTCAGCGATCTGTCTATTTCGTTCATCC
ATAGTTGCCTGACTCCCCGTCGTGTAGATAACTACGATACGGGAGGGCTTACCATCT
GGCCCCAGTGCTGCAATGATACCGCGAGACCCACGCTCACCGGCTCCAGATTTATC
AGCAATAAACCAGCCAGCCGGAAGGGCCGAGCGCAGAAGTGGTCCTGCAACTTTA
TCCGCCTCCATCCAGTCTATTAATTGTTGCCGGGAAGCTAGAGTAAGTAGTTCGCC
AGTTAATAGTTTGCGCAACGTTGTTGCCATTGCTACAGGCATCGTGGTGTCACGCT
CGTCGTTTGGTATGGCTTCATTCAGCTCCGGTTCCCAACGATCAAGGCGAGTTACA
TGATCCCCCATGTTGTGCAAAAAAGCGGTTAGCTCCTTCGGTCCTCCGATCGTTGT
CAGAAGTAAGTTGGCCGCAGTGTTATCACTCATGGTTATGGCAGCACTGCATAATT
CTCTTACTGTCATGCCATCCGTAAGATGCTTTTCTGTGACTGGTGAGTACTCAACCA
AGTCATTCTGAGAATAGTGTATGCGGCGACCGAGTTGCTCTTGCCCGGCGTCAATA
CGGGATAATACCGCGCCACATAGCAGAACTTTAAAAGTGCTCATCATTGGAAAACG
TTCTTCGGGGCGAAAACTCTCAAGGATCTTACCGCTGTTGAGATCCAGTTCGATGT
AACCCACTCGTGCACCCAACTGATCTTCAGCATCTTTTACTTTCACCAGCGTTTCTG
GGTGAGCAAAAACAGGAAGGCAAAATGCCGCAAAAAAGGGAATAAGGGCGACA

CGGAAATGTTGAATACTCATACTCTTCCTTTTTCAATATTATTGAAGCATTTATCAGG
GTTATTGTCTCATGAGCGGATACATATTTGAATGTATTTAGAAAAATAAACAAATAG
GGGTTCCGCGCACATTTCCCCGAAAAGTGCCACCTAAATTGTAAGCGTTAATATTTT
GTTAAAATTCGCGTTAAATTTTTGTTAAATCAGCTCATTTTTTAACCAATAGGCCGA
AATCGGCAAAATCCCTTATAAATCAAAAGAATAGACCGAGATAGGGTTGAGTGTTG
TTCCAGTTTGGAACAAGAGTCCACTATTAAAGAACGTGGACTCCAACGTCAAAGG
GCGAAAAACCGTCTATCAGGGCGATGGCCCACTACGTGAACCATCACCCTAATCAA
GTTTTTTGGGGTCGAGGTGCCGTAAAGCACTAAATCGGAACCCTAAAGGGAGCCC
CCGATTTAGAGCTTGACGGGGAAAGCCGGCGAACGTGGCGAGAAAGGAAGGGAA
GAAAGCGAAAGGAGCGGGCGCTAGGGCGCTGGCAAGTGTAGCGGTCACGCTGCG
CGTAACCACCACACCCGCCGCGCTTAATGCGCCGCTACAGGGCGCGTCCCATTCGC
CATTCAGGCTGCGCAACTGTTGGGAAGGGCGATCGGTGCGGGCCTCTTCGCTATTA
CGCCAGCTGGCGAAAGGGGGATGTGCTGCAAGGCGATTAAGTTGGGTAACGCCAG
GGTTTTCCCAGTCACGACGTTGTAAAACGACGGCCAGTGAATTGTAATACGACTCA
CTATAGGGCGAATTGGGTACCTTAACCCTAGAAAGATAGTCTGCGTAAAATTGACG
CATGCATTCTTGAAATATTGCTCTCTCTTTCTAAATAGCGCGAATCCGTCGCTGTGC
ATTTAGGACATCTCAGTCGCCGCTTGGAGCTCCCGTGAGGCGTGCTTGTCAATGCG
GTAAGTGTCACTGATTTTGAACTATAACGACCGCGTGAGTCAAAATGACGCATGAT
TATCTTTTACGTGACTTTTAAGATTTAACTCATACGATAATTATATTGTTATTTCATGT
TCTACTTACGTGATAACTTATTATATATATATTTTCTTGTTATAGATATCCAGACATGAT
AAGATACATTGATGAGTTTGGACAAACCACAACTAGAATGCAGTGAAAAAAATGC
TTTATTTGTGAAATTTGTGATGCTATTGCTTTATTTGTAACCATTATAAGCTGCAATA
AACAAGTTCTATCAGGCACCGGGCTTGCGGGTCATGCACCAGGTGCGCGGTCCTT
CGGGCACCTCGACGTCGGCGGTGACGGTGAAGCCGAGCCGCTCGTAGAAGGGGA
GGTTGCGGGGCGCGGAGGTCTCCAGGAAGGCGGGCACCCCGGCGCGCTCGGCCG
CCTCCACTCCGGGGAGCACGACGGCGCTGCCCAGACCCTTGCCCTGGTGGTCGGG
CGAGACGCCGACGGTGGCCAGGAACCACGCGGGCTCCTTGGGCCGGTGCGGCGC
CAGGAGGCCTTCCATCTGTTGCTGCGCGGCCAGCCGGGAACCGCTCAACTCGGCC
ATGCGCGGGCCGATCTCGGCGAACACCGCCCCCGCTTCGACGCTCTCCGGCGTGG
TCCAGACCGCCACCGCGGCGCCGTCGTCCGCGACCCACACCTTGCCGATGTCGAG

CCCGACGCGCGTGAGGAAGAGTTCTTGCAGCTCGGTGACCCGCTCGATGTGGCGG
TCCGGGTCGACGGTGTGGCGCGTGGCGGGGTAGTCGGCGAACGCGGCGGCGAGG
GTGCGTACGGCCCGGGGGACGTCGTCGCGGGTGGCGAGGCGCACCGTGGGCTTGT
ACTCGGTCATGAATTCCGAAAGGCCCGGAGATGAGGAAGAGGAGAACAGCGCGG
CAGACGTGCGCTTTTGAAGCGTGCAGAATGCCGGGCCTCCGGAGGACCTTCGGGC
GCCCGCCCCGCCCCTGAGCCCGCCCCTGAGCCCGCCCCCGGACCCACCCCTTCCC
AGCCTCTGAGCCCAGAAAGCGAAGGAGCAAAGCTGCTATTGGCCGCTGCCCCAA
AGGCCTACCCGCTTCCATTGCTCAGCGGTGCTGTCCATCTGCACGAGACTAGTGAG
ACGTGCTACTTCCATTTGTCACGTCCTGCACGACGCGAGCTGCGGGGCGGGGGGG
AACTTCCTGACTAGGGGAGGAGTAGAAGGTGGCGCGAAGGGGCCACCAAAGAAC
GGAGCCGGTTGGCGCCTACCGGGGGATGTGGAATGTGTGCGAGGCCAGAGGCCA
CTTGTGTAGCGCCAAGTGCCCAGCGGGGCTGCTAAAGCGCATGCTCCAGACTGCC
TTGGGAAAAGCGCCTCCCCTACCCGGTAGATATCATAACTTCGTATAATGTATGCTAT
ACGAAGTTATGCGGCCCTCGAGGTCGACATTGATTATTGACTAGTTATTAATAGTAA
TCAATTACGGGGTCATTAGTTCATAGCCCATATATGGAGTTCCGCGTTACATAACTTA
CGGTAAATGGCCCGCCTGGCTGACCGCCCAACGACCCCCGCCCATTGACGTCAATA
ATGACGTATGTTCCCATAGTAACGCCAATAGGGACTTTCCATTGACGTCAATGGGTG
GACTATTTACGGTAAACTGCCCACTTGGCAGTACATCAAGTGTATCATATGCCAAGT
ACGCCCCCTATTGACGTCAATGACGGTAAATGGCCCGCCTGGCATTATGCCCAGTA
CATGACCTTATGGGACTTTCCTACTTGGCAGTACATCTACGTATTAGTCATCGCTATT
ACCATGGGTCGAGGTGAGCCCCACGTTCTGCTTCACTCTCCCCATCTCCCCCCCCT
CCCCACCCCCAATTTTGTATTTATTTATTTTTTAATTATTTTGTGCAGCGATGGGGGC
GGGGGGGGGGGGGGCGCGCGCCAGGCGGGGCGGGGCGGGGCGAGGGGCGGGGC
GGGGCGAGGCGGAGAGGTGCGGCGGCAGCCAATCAGAGCGGCGCGCTCCGAAAG
TTTCCTTTTATGGCGAGGCGGCGGCGGCGGCGGCCCTATAAAAAGCGAAGCGCGC
GGCGGGCGGGAGTCGCTGCGTTGCCTTCGCCCCGTGCCCCGCTCCGCGCCGCCTC
GCGCCGCCCGCCCCGGCTCTGACTGACCGCGTTACTCCCACAGGTGAGCGGGCGG
GACGGCCCTTCTCCTCCGGGCTGTAATTAGCGCTTGGTTTAATGACGGCTCGTTTCT
TTTCTGTGGCTGCGTGAAAGCCTTAAAGGGCTCCGGGAGGGCCCTTTGTGCGGGG
GGGAGCGGCTCGGGGGGTGCGTGCGTGTGTGTGTGCGTGGGGAGCGCCGCGTGC

GGCCCGCGCTGCCCGGCGGCTGTGAGCGCTGCGGGCGCGGCGCGGGGCTTTGTGC
GCTCCGCGTGTGCGCGAGGGGAGCGCGGCCGGGGGCGGTGCCCCGCGGTGCGGG
GGGGCTGCGAGGGGAACAAAGGCTGCGTGCGGGGTGTGTGCGTGGGGGGGTGAG
CAGGGGGTGTGGGCGCGGCGGTCGGGCTGTAACCCCCCCCTGCACCCCCCTCCCC
GAGTTGCTGAGCACGGCCCGGCTTCGGGTGCGGGGCTCCGTGCGGGGCGTGGCG
CGGGGCTCGCCGTGCCGGGCGGGGGGTGGCGGCAGGTGGGGGTGCCGGGCGGGG
CGGGGCCGCCTCGGGCCGGGGAGGGCTCGGGGGAGGGGCGCGGCGGCCCCGGA
GCGCCGGCGGCTGTCGAGGCGCGGCGAGCCGCAGCCATTGCCTTTTATGGTAATCG
TGCGAGAGGGCGCAGGGACTTCCTTTGTCCCAAATCTGGCGGAGCCGAAATCTGG
GAGGCGCCGCCGCACCCCCTCTAGCGGGCGCGGGCGAAGCGGTGCGGCGCCGGC
AGGAAGGAAATGGGCGGGGAGGGCCTTCGTGCGTCGCCGCGCCGCCGTCCCCTTC
TCCATCTCCAGCCTCGGGGCTGCCGCAGGGGGACGGCTGCCTTCGGGGGGGACGG
GGCAGGGCGGGGTTCGGCTTCTGGCGTGTGACCGGCGGCTCTAGAGCCTCTGCTA
ACCATGTTCATGCCTTCTTCTTTTTCCTACAGCTCCTGGGCAACGTGCTGGTTGTTG
TGCTGTCTCATCATTTTGGCAAAGAATTCCTCGACGGTATCGATAAGCTATCACAAG
TTTGTACATTCGAAGGATCCACGCGTGCCACCATGGCCCTGCCTGTGACCGCCCTG
CTGCTGCCCCTGGCCCTGCTGCTGCATGCCGCCAGGCCTGATATTCAGATGACACA
GTCTCCTTCATCCCTGAGCGCTTCTGTGGGCGATAGAGTGACAATTACCTGTAAGG
CCAGTCAGAATGTGGGCACCAATGTGGCCTGGTACCAGCAGAAGCCCGGACAGA
GCCCTAAGGCCCTGATCTATAGCGCCAGCTACAGGGACAGCGGCGTGCCTGACAG
GTTTTCCGGCAGCGGCAGCGGCACCGACTTCACCCTGACCATTTCTTCCCTGCAGC
CTGAGGATTTCGCCACCTATTACTGCCAGCAGTACAAGAGCTATCCCCTGACCTTT
GGCCAGGGGACCCGGCTGGAAATCAAGGGCGGCGGCGGCAGCGGAGGGGGCGG
CTCCGGGGGCGGCGGCAGCCAGGTGCAGCTGCAGGAGAGCGGGCCTGGCCTGGT
GAAGCCCTCTGAGACCCTGAGCCTGACATGCACCGTGAGCGGGTACTCCATTACCT
CCGACTACGCCTGGAACTGGATCAGACAGCCTCCCGGCAAGGGCCTGGAGTGGAT
GGGCTACATCAGCTACAGCGGCAGCGCCACCTACAGCCCCTCTCTGAAGTCTCGG
ATTACCATCTCCAGGGACACATCCAAGAACCAGTTTAGCCTGAAGCTGTCTAGCGT
GACAGCCGCCGATACCGCCGTGTACTACTGTGCCAGAGGCGGCATCGCCGGCAGA
GGCAGGTGGGGCGCCATGGACTACTGGGGACAGGGCACCACCGTGACCGTGAGC

TCCACAACCACCCCAGCCCCAAGGCCTCCCACACCCGCTCCCACCATCGCCTCCC
AGCCCCTGAGCCTGAGACCAGAGGCCTGCAGACCCGCCGCCGGCGGCGCCGTGC
ACACCAGAGGCCTGGACTTCGCCTGCGATTCCAACCTGTTCGTGGCCTCCTGGATC
GCCGTGATGATTATCTTTCGGATCGGCATGGCCGTGGCCATCTTTTGTTGCTTTTTCT
TCCCCTCTTGGCGCCGCAAGAGGAAGGAGAAGCAGTCCGAGACCAGCCCCAAGG
AGTTCCTGACCATCTACGAGGATGTGAAGGACCTGAAAACCCGGAGGAATCACGA
GCAGGAGCAGACATTCCCCGGCGGCGGCTCCACCATCTACAGCATGATCCAGAGC
CAGAGCAGCGCCCCCACCTCTCAGGAGCCCGCCTACACTCTGTACTCTCTGATCCA
GCCCAGCAGAAAGTCCGGCTCCAGAAAGAGAAACCATTCTCCATCTTTTAATAGTA
CAATCTATGAAGTGATTGGAAAAAGCCAGCCAAAGGCCCAGAACCCTGCTCGTCT
GTCTAGAAAAGAATTAGAGAATTTTGATGTGTATAGCAGAGTGAAATTCTCTAGGT
CTGCTGACGCTCCTGCCTATAAGCAGGGCCAGAATCAGCTGTATAATGAGCTGAAT
CTGGGAAGGAGAGAAGAATATGATGTGCTGGATAAAAGAAGGGGCAGAGATCCTG
AGATGGGCGGAAAACCTAGAAGAAAGAATCCTCAGGAAGGCCTGTATAATGAACT
GCAGAAGGATAAAATGGCTGAAGCCTATAGCGAGATTGGCATGAAAGGCGAAAGA
AGAAGAGGCAAGGGCCATGATGGCCTGTATCAGGGACTGAGTACAGCCACTAAAG
ATACATATGACGCCCTGCACATGCAGGCCCTGCCACCTAGATGAATGCATGCTAGC
CTCGACTGTGCCTTCTAGTTGCCAGCCATCTGTTGTTTGCCCCTCCCCCGTGCCTTC
CTTGACCCTGGAAGGTGCCACTCCCACTGTCCTTTCCTAATAAAATGAGGAAATTG
CATCGCATTGTCTGAGTAGGTGTCATTCTATTCTGGGGGGTGGGGTGGGGCAGGAC
AGCAAGGGGGAGGATTGGGAAGACAATAGCAGGCATGCTGGGGATGCGGTGGGC
TCTAT (SEQ ID NO: 17).

### Example 3: Differentiation of iPSCs Expressing Anti-GPRC5D CAR Polypeptide into CAR-iNK Cells

FIG. 4 shows a general workflow for obtaining gene-edited iPSC-derived anti-GPRC5D CAR-iNK cells. The iPSCs expressing the anti-GPRC5D CAR polypeptide obtained in Examples 2 and 3 were differentiated into CAR-iNK cells through the following specific steps.
1) When the confluence of iPSCs reached 75% to 85%, the cells were washed once with DMEM/F12 medium and digested with the Accutase enzyme or TrypLE enzyme in a 37°C incubator with 5% CO₂. Subsequently, the cells were centrifuged and the supernatant was discarded. The cells were then resuspended in mTeSR^{™}1 medium supplemented with 5 µM Y27632, 50 ng/mL BMP4, and 10 µM CHIR99021 to obtain a single-cell suspension of iPSCs.
2) An appropriate number of the single-cell suspension of iPSCs was transferred into AggreWell^{™} microplates, in accordance with the instructions for AggreWell^{™} 400 or AggreWell^{™} 800, to ensure that the single cells seeded in each well were evenly distributed throughout the well. The cell-loaded microplate was centrifuged at 100 g for 3 minutes and cultured statically at 37°C, 5% CO₂, and 95% humidity for 1 day to 2 days. Subsequently, the culture medium was replaced with the second differentiation medium, and then the medium was changed every 2 days, with continuous culture for 4 days to 5 days. The second differentiation medium included: StemPro-34 complete medium supplemented with 1% ITS-X, 1 µM β-mercaptoethanol, 50 ug/mL ascorbic acid, 1x GlutaMAX, 50 ng/mL VEGF, 50 ng/mL bFGF, 50 ng/mL BMP4, and 35 nM UM171.
3) Subsequently, the medium was replaced with the third differentiation medium. The third differentiation medium included: StemPro-3^{™} SFM complete medium supplemented with 1% ITS-X, 1 µM β-mercaptoethanol, 50 µg/mL ascorbic acid, 1x GlutaMAX, 50 ng/mL VEGF, 50 ng/mL bFGF, 50 ng/mL SCF, 10 ng/mL FLT3L, and 30 ng/mL TPO.
4) After the above three stages of culture, generally on day 10 to day 14 post-differentiation, depending on the CD34⁺ FACS results that when the proportion of CD34⁺ cells exceeded 80%, the medium was replaced with the fourth differentiation medium for further differentiation into NK cells. This process generally lasted for 3 weeks to 5 weeks. The fourth differentiation medium included: DMEM/F12, 15% human AB serum, 5 ng/mL IL-3 (used in the first week), 20 ng/mL IL-7, 10 ng/mL IL-15, 10 ng/mL FLT3L, and 50 ng/mL SCF. In some cases, the above CAR-iNK cells may be stimulated by irradiated aAPCs (K562 cells expressing membrane-bound IL-21 and 4-1BBL) and further expanded in the amplification medium to achieve sufficient cell numbers. The amplification medium included GT-T551 H3 medium supplemented with 5% FBS, 1000 IU/mL IL-2, and 10 ng/mL IL-15.

All the antibodies (including isotype controls and secondary antibodies) were purchased from Biolegend except that the anti-GPRC5D scFv antibody was sourced from Kangyuan Bochuang. FACS detection procedures may be performed in accordance with in the standard operating protocols commonly used for the relevant instruments. The FACS detection results of expression levels of CD56, CD45, NKG2D, TCRαβ, and TCRY*δ* in GPRC5D CAR-iNK cells are illustrated in FIG. 6 to FIG. 8. The results show that the anti-GPRC5D CAR exerted no adverse effects on the differentiation and amplification of the anti-GPRC5D CAR-iNK cells. The expression levels of NK-related markers were similar between the anti-GPRC5D CAR-iNK cells and the WT iNK cells, and the expression level of NKG2D (an activation receptor of the NK cell) in the anti-GPRC5D CAR-iNK cells was superior to that in WT iNK cells.

### Example 4: Determination of Killing activity of NK Cells Against Tumor Cells Using the Luciferase Method

Using K562-Luc tumor cells as target cells and using anti-GPRC5D CAR-iNK, CBNK, WT iNK, and anti-BCMA CAR-iNK cells as effector cells, the killing activity of NK cells against target cells was detected at an effector-to-target ratio of 4:1. The specific experimental procedures are as follows:

A K562-Luc target cell suspension was prepared (the target cell viability not less than 90%). After counting, the cells were seeded into the wells of a 96-well plate according to a specified effector-to-target ratio (a fixed effector-to-target ratio of 4:1), and three replicate wells were arranged for each effector cell at the corresponding effector-to-target ratio. The medium in each well was adjusted to 100 µL, followed by the addition of the required number of effector cells, namely anti-GPRC5D CAR-iNK, CBNK, WT iNK and anti-BCMA CAR-iNK cells, according to the specified effector-to-target ratio. The 96-well plate was placed in a 37°C incubator with 5% CO₂ and incubated for a predetermined duration (e.g., 4 h). A luciferin substrate was diluted 100-fold in cell culture medium. 50 µL of diluted luciferin substrate solution was added to each well and incubated at room temperature in the dark for 10 minutes. The microplate reader was set to Lum with a duration of 1000 ms, followed by measurement on the instrument. Killing efficiency % = (control group - killing group) / cell control group * 100. WT iNK refers to iNK cells differentiated from WT iPSC. The preparation process of anti-BCMA CAR-iNK cells or anti-GPRC5D/BCMA CAR-iNK cells was similar to that of the above-described anti-GPRC5D CAR-iNK cells, except that the GPRC5D antibody in the anti-GPRC5D CAR polypeptide was replaced with a BCMA antibody (BCMA sdAb, the amino acid sequence as set forth in SEQ ID NO: 24), or a GPRC5D/BCMA bispecific antibody (the amino acid sequence as set forth in SEQ ID NO: 10).

QVKLEESGGGLVQAGRSLRLSCAASEHTFSSHVMGWFRQAPGKERESVAV
IGWRDISTSYADSVKGRFTISRDNAKKTLYLQMNSLKPEDTAVYYCAARRIDAADFDS
WGQGTQVTVSSGGGGSGGGGSGGGGSEVQLVESGGGLVQAGGSLRLSCAASGRTFT
MGWFRQAPGKEREFVAAISLSPTLAYYAESVKGRFTISRDNAKNTVVLQMNSLKPED
TALYYCAADRKSVMSIRPDYWGQGTQVTVSS (SEQ ID NO: 24).

The results, as illustrated in FIG. 9A, show that different effector cells exhibited similar killing activities against K562-Luc. Since K562-Luc did not express GPRC5D or BCMA, FIG. 9A showed that these effector cells exhibited similar basal killing activities against the target cells.

### Example 5: Determination of Basal Killing Activity of Effector Cells against Target Cells

Using the tumor cells K562-Luc as the target cells and using anti-GPRC5D CAR-iNK, WT iNK, and anti-BCMA CAR-iNK cells as the effector cells, the cells were mixed at different effector-to-target ratios (4:1, 2:1, or 1:1), and the killing activities of the NK cells against K562-Luc cells were determined in accordance with the method described in Example 4. FIG. 9B show that the basal killing activities of WT iNK, anti-GPRC5D CAR-iNK, and anti-BCMA CAR-iNK against the target cells K562-Luc exhibited dose-dependent effects.

### Example 6: Anti-GPRC5D CAR Molecules Enhance Killing Activity of iNK Cells Against GPRC5D⁺ Tumor Cells

The effector cells (CBNK, WT iNK, GPRC5D CAR-iNK, anti-BCMA CAR-iNK, and anti-GPRC5D/BCMA CAR-iNK) were mixed with the target cells NCI-H929-Luc2 cells (hereinafter referred to as H929-Luc2) that highly expressed GPRC5D and BCMA at a fixed effector-to-target ratio of 4:1 and different effector-to-target ratios (8:1, 4:1, or 1:1), respectively. Unless otherwise specified, the cells were co-cultured at 37°C with 5% CO₂ for 4 hours. The killing activities of iNK cells in different groups against the target cells H929-Luc2 were measured using the method provided in Example 4.

The results, as illustrated in FIG. 10A, show that under conditions of an effector-to-target ratio of 4:1 and co-incubation for 4 hours, CBNK cells were insensitive to H929-Luc2 cells and exhibited almost no killing activities; WT iNK cells can kill approximately 10% of H929-Luc2 cells; whereas anti-GPRC5D CAR-iNK cells can kill approximately 90% of H929-Luc2 cells. FIG. 10B show that the killing activities of WT iNK and anti-GPRC5D CAR-iNK cells against the H929-Luc2 cells exhibited dose-dependent effects, which decreased as the effector-to-target ratio declined.

The killing activities of the anti-GPRC5D CAR-iNK, anti-BCMA CAR-iNK, and anti-GPRC5D/BCMA CAR-iNK cells against the H929-Luc2 cells were further compared. The experimental method was also referred to Example 4 described above. The results, as illustrated in FIG. 11, show that at the same effector-to-target ratio, the killing activities of the anti-GPRC5D CAR-iNK cells were stronger than those of the anti-BCMA CAR-iNK cells. In addition, the anti-GPRC5D/BCMA CAR-iNK cells exhibited better killing activities at low effector-to-target ratios.

In summary, the expression of the anti-GPRC5D CAR molecules can endow the NK cells with far superior killing activities against GPRC5D⁺ cells compared with WT iNK cells or CBNK cells. In addition, the iNK cells expressing the anti-GPRC5D CAR molecules exhibited better killing activities against MM cell line H929-Luc2 than those expressing the anti-BCMA CAR molecules. Also, the killing activities of the anti-GPRC5D CAR molecules against H929-Luc2 can be further enhanced by the addition of BCMA antigen recognition domain. Furthermore, after loading the anti-GPRC5D or anti-BCMA chimeric antigen receptor at the iPSC stage, these iPSCs were further differentiated into anti-GPRC5D or anti-BCMA CAR-iNK cells, which can specifically kill the target cells expressing GPRC5D or BCMA.

### Example 7: Anti-GPRC5D CAR Molecules Enhance Killing Activity of Cryopreserved iNK Cells against GPRC5D⁺ Tumor Cells

The effector cells in this example were the iNK cells (anti-GPRC5D CAR-iNK and WT iNK) differentiated in Example 3, and the target cells were K562-Luc. The killing activity of the cryopreserved iNK cells against the tumor cells after thawing was detected.

First, the iNK cells (anti-GPRC5D CAR-iNK and WT iNK) differentiated in Example 3 were thawed. The detailed experimental procedures were carried out as follows. The water bath/metal bath was preheated and maintained at a stable temperature of 37°C in advance. 9 mL of the pre-chilled iNK cell culture medium was added into a new centrifuge tube using a pipette. The cryovial was taken out of the liquid nitrogen, gently rubbed between the hands, and then quickly transferred to the 37°C water bath or metal bath. The tube was continuously shaken until only a small piece of ice remained in the cryovial. After being passed through the pass-through window, the tube was disinfected with alcohol and placed in a biosafety cabinet. The cell cryovial was gently opened, and the cells were gently transferred into the centrifuge tube containing the culture medium using a pipette, followed by thorough mixing. The cell suspension was then centrifuged (300 g*5 min), and the supernatant was discarded. 10 mL of NK culture medium was added for resuspension and mixing, followed by cell counting. Subsequently, centrifugation was performed and the supernatant was discarded. According to the total cell count, the NK cell culture medium was supplemented to adjust the cell density to 1×10⁶ cells/mL. After thorough mixing, the cell suspension was seeded into culture flasks or plates, or immediately used for cell killing assays.

The iNK cells were harvested within 72 hours of thawing, and their killing activities against the target cells K562-Luc were measured using the method in Example 4. The results, as illustrated in FIG. 12A, show that both the WT iNK and anti-GPRC5D CAR-iNK cells after thawing exhibited dose-dependent effects in their basal killing activities, and the anti-GPRC5D CAR-iNK cells exhibited a slightly higher basal killing activity than the WT iNK cells. However, FIG. 12B shows that the thawed WT iNK cells were completely deprived of their killing activity against H929-Luc2, whereas the thawed anti-GPRC5D CAR-iNK cells still exhibited good killing activity against H929-Luc2.

By comparing the basal killing activity (against K562-Luc) and specific killing activity (against H929-Luc2) of the anti-GPRC5D CAR-iNK cells before and after cryopreservation and thawing, the results show that the cryopreservation and thawing process exerted a greater impact on the basal killing activity of the anti-GPRC5D CAR-iNK cells, while it had a minimal effect on their specific killing activity (illustrated in FIG. 13A and FIG. 13B).

The killing activities of WT iNK and anti-GPRC5D CAR-iNK cells after cryopreservation and thawing against other multiple myeloma cell lines were further tested. The results, as illustrated in FIG. 14, show that regardless of target cells with high GPRC5D expression (MM.1S-Luc2) or with low GPRC5D expression (OPM-2-Luc2), the anti-GPRC5D CAR-iNK cells after cryopreservation and thawing exhibited highly efficient killing activity against these target cells. In contrast, the WT iNK cells only exhibited killing activity against MM.1S-Luc2 at high effector-to-target ratios (Effector:Target = 8:1, with approximately 40% killing activity; Effector:Target = 4:1, with approximately 20% killing activity).

The above results indicate that the cryopreservation and thawing process exerted a minimal effect on the specific killing activity of the anti-GPRC5D CAR-iNK cells, and the anti-GPRC5D CAR-iNK cells exhibited good killing activity against both the target cells with high GPRC5D expression and the target cells with low GPRC5D expression.

### Example 8: Optimization of Anti-GPRC5D CAR Molecules

In NK cells, the NKG2D transmembrane sequence played a crucial role in the specific recognition function of CAR molecules (https://doi.org/10.1016/j.stem.2018.06.002). To further enhance the function of CAR-iNK cells, the inventors mutated some sites based on the NKG2D transmembrane sequence (SEQ ID NO: 18; TM control), and designed the following NKG2D transmembrane sequence variants (see Table 1, with mutated amino acid sites underlined), which were used as the transmembrane domains of CAR molecules. The transmembrane domain (SEQ ID NO: 18) in the amino acid sequence of the anti-GPRC5D CAR (SEQ ID NO: 15) was replaced with the transmembrane sequences shown in Table 1, obtaining fourteen anti-GPRC5D CAR molecule variants (CAR-TM1 to CAR-TM14; hereinafter referred to as TM1, TM2, TM3, TM4, TM4, TM5, ..., TM14). Using the method in Example 2, transposon plasmids expressing the above fourteen anti-GPRC5D CAR molecules with transmembrane sequence variants were obtained. 2.5 µg of transposon plasmids prepared above were transfected into 293T cells, respectively, using jetOPTIMUS^{®} DNA transfection reagent. After 24 hours, the expression of the anti-GPRC5D scFV in the CAR molecules was detected by flow cytometry. The antibody information is provided in Example 2. The results, as illustrated in FIG. 15, show that the expression level of the CAR molecule containing TM1 was similar to that of the control CAR molecule (TM control); and the expression level of the CAR molecule containing TM4, TM5, TM6, or TM8 was slightly higher than that of the control CAR molecule (TM control). The effects of TM9 to TM14 were not further investigated in subsequent experiments.

Further, using the method in Example 2, the transposon plasmids containing CAR molecules with TM control and TM1 to TM8 and PBase plasmids were introduced into iPSCs to obtain iPSCs expressing CAR molecules and variants thereof. Using the method in Example 3, the above-described iPSCs expressing exogenous anti-GPRC5D CAR were differentiated into NK cells. iHSPCs were obtained on day 10 of differentiation. The CD34 expression level of the iHSPC containing TM6 CAR molecule was slightly lower, whereas the CD34 expression levels of all other iHSPCs exceeded 70% (FIG. 16). The CD43 expression levels were similar across all iHSPCs, with each exceeding 97% (FIG. 16). The above-described iHSPCs were further differentiated until day 23, obtaining NK cells expressing CD314 and CD56 (Day 23 CAR-iNK). Interestingly, the expression levels of CD314 and CD56 in all CAR-iNK cells containing transmembrane sequence variants were higher than those in the control group (TM control) (FIG. 17). This indicates that the use of CAR molecules containing the transmembrane sequence variants did not affect the differentiation of iPSCs into NK cells, but may promote their differentiation to varying degrees. In addition, at the iHSPC stage, the CD34 expression level of CAR carrying TM6 was low (50.18%), but it did not affect their further differentiation into NK cells (FIG. 17). Subsequently, the proliferation of Day 23 CD56⁺ CAR-iNK cells relative to the number of iPSCs in the initial differentiation stage was further quantified. The results show that CD56⁺ CAR-iNK cells containing TM variants exhibited similar or even higher proliferation fold changes relative to the control cells. Among which, the proliferation fold change of the TM5 group exceeded 100 times that of the control group; the proliferation fold change of the TM4, TM7 and TM8 groups exceeded 10 times that of the control group; and the proliferation fold change of the TM6 group reached 7 times that of the control group (FIG. 18).

Furthermore, the inventors expanded the Day 23 CAR-iNK cells to 36 days to obtain mature CAR-iNK cells. The CD45 expression level of cells in all groups exceeded 96% (except for the TM2 group, all others exceeding 99.6%), the CD56 expression level of cells in all groups exceeded 90% (the TM5 and TM8 groups exceeding 99%), and the CAR molecule expression level of cells in all groups was above 95% (measured based on the expression level of anti-GPRC5D scFV; FIG. 19, i.e., FIG. 19A, FIG. 19B, FIG. 19C, and FIG. 19D). These cells exhibited a phenotype of high-purity mature NK cells, and the CAR molecule expression levels were similar between the TM variant group and the TM control group.

To further verify the effect of CAR molecules carrying TM mutants on the antigen-dependent specific killing function of iNK cells, the method of Example 4 was used. K562-Luc cells and K562-GPRC5D-Luc cells (K562-Luc cell line expressing the exogenous GPRC5D antigen; cells sourced from Cell Inspire Biotechnology Co., Ltd., and constructed by overexpressing the GPRC5D protein in K562 cells via the lentiviral method) were used as target cells. These target cells were co-cultured with CAR-iNK cells carrying CAR molecules with TM control or TM variant at an effector-to-target ratio of 1:1 for 4 hours to determine the killing activities of these CAR-iNK cells against the target cells. Since the iNK cells exhibit a broad-spectrum killing activity, they would exhibit a certain degree of killing activity against K562-Luc cells. The CAR molecules carrying anti-GPRC5D can further enhance the antigen-dependent specific killing activity of iNK cells against GPRC5D⁺ target cells. Accordingly, in this experiment, the killing activities of CAR-iNK cells in each group against K562-Luc and K562-GPRC5D-Luc were measured, respectively. The antigen-dependent specific killing activity of anti-GPRC5D CAR-iNK cells obtained from the expression of the CAR molecules was calculated by subtracting the killing activity of CAR-iNK cells against K562-Luc cells from the killing activity of CAR-iNK cells against K562-GPRC5D-Luc cells. The results, as illustrated in FIG. 20, show that the TM5, TM6, and TM8 groups exhibited a significantly enhanced antigen-dependent specific killing activity against K562-GPRC5D-Luc compared with the TM control group; the TM1, TM4, and TM control groups exhibited a similar antigen-dependent specific killing activity against K562-GPRC5D-Luc; and the TM2, TM3 and TM7 groups exhibited no obvious antigen-dependent specific killing activity against K562-GPRC5D-Luc.

In summary, compared with CAR molecules carrying the reverse NKG2D transmembrane domain (NKG2D-TM control, or abbreviated as TM control), CAR molecules carrying TM5, TM6, and TM8 variants can endow NK cells with superior antigen-dependent specific killing activities; and CAR molecules carrying TM1 and TM4 variants can enable NK cells to exhibit similar antigen-dependent specific killing activities. In addition, TM1 to TM8 variants did not affect the differentiation and proliferation of iPSCs into NK cells, and CAR molecules carrying some TM variants (TM1 to TM8) promoted the differentiation and proliferation of the iPSCs into the NK cells. Among which TM5 exhibited the most pronounced effect (the proliferation fold change of CD56⁺ NK cells exceeding 100 times that of the control group), followed by TM4, TM7, and TM8 (the proliferation fold change of CD56⁺ NK cells exceeding 10 times that of the control group), and then TM6 (the proliferation fold change of CD56⁺ NK cells exceeding 7 times that of the control group). TM3, TM2, and TM1 exhibited the least pronounced effect, with the proliferation fold changes of CD56⁺ NK cells reaching 3.7 times, 3.5 times and 1.7 times that of the control group, respectively.

**Table 1: Transmembrane Region Sequences for CAR Molecules**

| Name | Transmembrane region sequences for CAR molecules | Sequence No. |
|---|---|---|
| NKG2D-TM Control | SNLFVASWIAVMIIFRIGMAVAIFCCFFFPS | SEQ ID NO: 18 |
| NKG2D-TM1 | SNLFVASWIAVMIIFRIGMAVAIFCCFFLPS | SEQ ID NO: 19 |
| NKG2D-TM2 | SNLFVASWIAVMVIFRIGMAVAIFRCLFFPS | SEQ ID NO: 20 |
| NKG2D-TM3 | SNLFVASWIAVMIIFRIGMAVAIFCCLFFPS | SEQ ID NO: 21 |
| NKG2D-TM4 | SNLFVASWIAVMIIFHIGLAIAISHVLFFPS | SEQ ID NO: 22 |
| NKG2D-TM5 | SNLFVASWIAVMIIFHIGMAIAISRCLFFPS | SEQ ID NO: 23 |
| NKG2D-TM6 | SNLFVASWIAVMIIFHIGMAIAISCTVFFPS | SEQ ID NO: 27 |
| NKG2D-TM7 | SNLFVASWIAVMIIFRIGMAVAVSRCLHFPS | SEQ ID NO: 28 |
| NKG2D-TM8 | SNLFVASWIAVMIIFHIGLAIAISRCLFYPS | SEQ ID NO: 29 |
| NKG2D-TM9 | SNLFVASWIAVMVIFRIGMAIAISRALFFPS | SEQ ID NO: 30 |
| NKG2D-TM10 | SNLFVASWIAVMVIFRVGMAVAISRALFFPS | SEQ ID NO: 31 |
| NKG2D-TM11 | SNLFVASWIAVMVIFRIAMAVAISRALFFPS | SEQ ID NO: 32 |
| NKG2D-TM12 | SNLFVASWIAVMVIFRIAMSIAIFRGLFFPS | SEQ ID NO: 33 |
| NKG2D-TM13 | SNLFVASWIAVMVTFRIGMAIAISRALFFPS | SEQ ID NO: 34 |
| NKG2D-TM14 | SNLFVASWIAVMVLFRIAMAIAIIYGLFFPS | SEQ ID NO: 35 |

Although the embodiments of the present disclosure have been illustrated and described above, it will be understood by those of ordinary skill in the art that the above embodiments are exemplary only and should not be construed as limiting the scope of the invention. Modifications, alterations, substitutions, and variations can be made to the described embodiments without departing from the scope of the present disclosure.

## Claims

1. A chimeric antigen receptor, comprising:
an extracellular domain, capable of binding to a tumor antigen, an inflammatory factor, or a pathogen antigen;
a transmembrane domain, linked to the extracellular domain, wherein the transmembrane domain is from a type I transmembrane protein or an artificially synthesized transmembrane protein with a hydrophobic helix; and
an intracellular domain, linked to the transmembrane domain,
wherein the amino acid sequence of the transmembrane domain has the following sequence:
SNLFVASWIAVMX₈IFX₉IGX₁₀AX₁₁AX₁₂X₁₃X₁₄X₁₅X₁₆X₁₇X₁₈X₁₉PS, where:
X₈ is selected from I or V;
X₉ is selected from R or H;
X₁₀ is selected from M or L;
X₁₁ is selected from V or I;
X₁₂ is selected from I or V;
X₁₃ is selected from S or F;
X₁₄ is selected from C, R, or H;
X₁₅ is selected from C, T, or V;
X₁₆ is selected from V, L, or absent;
X₁₇ is selected from F or H;
X₁₈ is selected from F or Y;
X₁₉ is selected from L or absent; and
X₁₆ and X₁₉ are not present simultaneously, and X₁₆ and X₁₉ are not absent simultaneously; and
wherein the tumor antigen is a tumor-specific antigen or a tumor-associated antigen.

2. The chimeric antigen receptor according to claim 1, wherein the transmembrane domain has the amino acid sequence as set forth in any one of SEQ ID NO: 19 to SEQ ID NO: 23 and SEQ ID NO: 27 to SEQ ID NO: 29.

3. The chimeric antigen receptor according to claim 1, wherein the extracellular domain comprises an antibody or antigen-binding fragment thereof capable of specifically recognizing at least one selected from the group consisting of: GPRC5D, CD19, BCMA, CD123, CD138, CD20, CD22, CD30, CD33, CD38, CD4, CD7, CLL-1, CD10, CD34, CS1, CD16, CD5, IL-1-RAP, ITGB7, k-IgG, TACI, TRBC1, MUC1, NKG2DL, PD-L1, CD133, CD117, LeY, CD70, ROR1, CD147, CD171, CD267, AXL, B7-H3, CD44v6, CEA, CAIX, CLDN18.2, CLDN6, DLL3, DR5, EGFR, EpCAM, ErbB, FAP, FBP, FRα, GD2, gp100, HER2, IL-13Rα2, LFA1, LMP1, MAGE-A3, MAGE-A1, MAGE-A4, MART1, MET, MG7, MMP2, MSLN, MUC1, MUC16, MUC16ecto, NECTIN4, NKG2D, PMEL, PSCA, PSMA, ROR1, ROR2, TM4SF1, TnMUC1, VEGFR2, CLD18, and GPC3;
optionally, the extracellular domain further comprises an antibody or antigen-binding fragment thereof capable of specifically recognizing a pathogen antigen, the pathogen comprising EBV;
optionally, the extracellular domain further comprises an antibody or antigen-binding fragment thereof capable of specifically recognizing an inflammatory factor, the inflammatory factor comprising at least one selected from the group consisting of: IL-1, TNF-α, IFN-γ, IL-12, IL-18, GMCSF, IL-6, IL-8, IL-17, CXCL1, CXCL2, CXCL9, CXCL10, CXCL11, CXCL16, and CCL2-2; and
preferably, the extracellular domain comprises an antibody or antigen-binding fragment thereof capable of specifically recognizing GPRC5D.

4. The chimeric antigen receptor according to claim 3, wherein the antibody or the antigen-binding fragment capable of specifically recognizing GPRC5D comprises at least one CDR sequence selected from the following or an amino acid sequence having at least 90% identity thereto:
heavy chain variable region CDR sequences: SEQ ID NO: 1 to SEQ ID NO:3; and
light chain variable region CDR sequences: SEQ ID NO: 4, the amino acid sequence SAS, and SEQ ID NO: 5;
optionally, the antibody or the antigen-binding fragment capable of specifically recognizing GPRC5D comprises:
heavy chain variable region CDR1, CDR2, and CDR3 sequences as set forth in SEQ ID NO: 1, SEQ ID NO: 2, and SEQ ID NO: 3, respectively, or amino acid sequences having at least 90% identity thereto;
optionally, the antibody or the antigen-binding fragment capable of specifically recognizing GPRC5D comprises:
light chain variable region CDR1, CDR2, and CDR3 sequences as set forth in SEQ ID NO: 4, the amino acid sequence SAS, and SEQ ID NO: 5, respectively, or amino acid sequences having at least 90% identity thereto;
optionally, the antibody or the antigen-binding fragment capable of specifically recognizing GPRC5D comprises:
heavy chain variable region CDR1, CDR2, and CDR3 sequences as set forth in SEQ ID NO: 1, SEQ ID NO: 2, and SEQ ID NO: 3, respectively, or amino acid sequences having at least 90% identity thereto; and
light chain variable region CDR1, CDR2, and CDR3 sequences as set forth in SEQ ID NO: 4, the amino acid sequence SAS, and SEQ ID NO: 5, respectively, or amino acid sequences having at least 90% identity thereto;
optionally, the antibody or the antigen-binding fragment capable of specifically recognizing GPRC5D comprises a heavy chain variable region having the amino acid sequence as set forth in SEQ ID NO: 6;
optionally, the antibody or the antigen-binding fragment capable of specifically recognizing GPRC5D comprises a light chain variable region having the amino acid sequence as set forth in SEQ ID NO: 7; and
optionally, the antibody capable of specifically recognizing GPRC5D is a single-chain antibody having the amino acid sequence as set forth in SEQ ID NO: 8.

5. The chimeric antigen receptor according to claim 4, wherein the extracellular domain comprises a bispecific binding molecule, the bispecific binding molecule comprising:
a first binding region, comprising the antibody or antigen-binding fragment thereof capable of specifically recognizing GPRC5D; and
a second binding region, having an activity of binding to an antigen specifically expressed on lymphocytes.

6. The chimeric antigen receptor according to claim 5, wherein the antigen specifically expressed on the lymphocytes comprises at least one selected from the group consisting of BCMA, CD38, CD138, CD3E, and FCGR3A;
optionally, the second binding region comprises a single-chain antibody or a single-domain antibody;
optionally, the second binding region has BCMA-binding activity;
optionally, the second binding region comprises a single-chain antibody or a single-domain antibody capable of specifically recognizing BCMA; and
optionally, the second binding region has the amino acid sequence as set forth in SEQ ID NO: 9.

7. The chimeric antigen receptor according to claim 5, wherein the first binding region is linked to the second binding region via a linking peptide;
optionally, the bispecific binding molecule has the amino acid sequence as set forth in SEQ ID NO: 10.

8. The chimeric antigen receptor according to claim 1, wherein the extracellular domain is linked to the transmembrane domain via a hinge domain;
optionally, the hinge domain is from a hinge region of at least one protein selected from the group consisting of: CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT;
optionally, the hinge domain is from a hinge region of CD8; and
optionally, the hinge domain has the amino acid sequence as set forth in SEQ ID NO: 11.

9. The chimeric antigen receptor according to claim 7 or 8, wherein,
the intracellular domain comprises a co-stimulatory signal domain and an intracellular signaling domain, the co-stimulatory signal domain being linked to the intracellular signaling domain via a linking peptide;
optionally, the co-stimulatory signal domain comprises an intracellular co-stimulatory signal domain of at least one protein selected from the group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88;
optionally, the co-stimulatory signal domain is from an intracellular co-stimulatory signal domain of 2B4;
optionally, the co-stimulatory signal domain has the amino acid sequence as set forth in SEQ ID NO: 12;
optionally, the intracellular signaling domain comprises at least one ITAM motif; optionally, the intracellular signaling domain comprises an intracellular signaling domain from at least one protein selected from the group consisting of: CD3ζ, CD3δ, CD3γ, CD3ε, CD79a, CD79b, FcεRIγ, FcεRIβ, FcγRIIa, DAP10, and DAP-12;
optionally, the intracellular signaling domain is an intracellular signaling domain from CD3ζ;
optionally, the intracellular signaling domain has the amino acid sequence as set forth in SEQ ID NO: 13;
optionally, the chimeric antigen receptor further comprises a signal peptide linked to the N-terminus of the extracellular domain of the chimeric antigen receptor;
optionally, the signal peptide is from CD8 protein;
optionally, the signal peptide has the amino acid sequence as set forth in SEQ ID NO: 14; optionally, the chimeric antigen receptor has the amino acid sequence as set forth in SEQ ID NO: 15 or SEQ ID NO:16.

10. An isolated polynucleotide, encoding the chimeric antigen receptor according to any one of claims 1 to 9.

11. An expression vector, carrying the polynucleotide according to claim 10.

12. A cell, carrying the polynucleotide according to claim 10 or the expression vector according to claim 11, or capable of expressing the chimeric antigen receptor according to any one of claims 1 to 9.

13. The cell according to claim 12, comprising an immune cell,
optionally, the immune cell being isolated from peripheral blood, umbilical cord blood, or tissues or organs, or is differentiated from a stem cell or an immune progenitor cell;
optionally, the stem cell comprising an induced pluripotent stem cell, a hematopoietic stem cell, or an embryonic stem cell within 14 days after fertilization and not subjected to *in vivo* development; and
optionally, the immune cell comprises at least one selected from the group consisting of an NK cell, a T cell, and a macrophage.

14. A composition, comprising at least one of the chimeric antigen receptor according to any one of claims 1 to 9, the polynucleotide according to claim 10, the expression vector according to claim 11, or the cell according to claim 12 or 13.

15. Use of the composition according to claim 14 in the manufacture of a medicament for treating a disease, wherein the disease is a hematologic malignancy or solid tumor, an autoimmune disease, or a viral infectious disease;
optionally, the hematologic malignancy or solid tumor is a GPRC5D-associated disease.

16. The use according to claim 15, wherein the GPRC5D-associated disease comprises multiple myeloma.

17. A kit for detecting GPRC5D, the kit comprising the chimeric antigen receptor according to any one of claims 1 to 9.

18. Use of the chimeric antigen receptor according to any one of claims 1 to 9, the polynucleotide according to claim 10, or the expression vector according to claim 11 in the manufacture of a kit for detecting GPRC5D or diagnosing a GPRC5D-associated disease.

19. The use according to claim 18, wherein the GPRC5D-associated disease comprises multiple myeloma.

20. Use of the chimeric antigen receptor according to any one of claims 1 to 9, the polynucleotide according to claim 10, the expression vector according to claim 11, the cell according to claim 12 or 13, and/or the composition according to claim 14 in the treatment of a hematologic malignancy or solid tumor, an autoimmune disease, or a viral infectious disease in a subject.

21. A method for preventing and/or treating a hematologic malignancy or solid tumor, an autoimmune disease, or a viral infectious disease in a subject, the method comprising:
administering to the subject at least one of the chimeric antigen receptor according to any one of claims 1 to 9, the polynucleotide according to claim 10, the expression vector according to claim 11, the cell according to claim 12 or 13, or the composition according to claim 14.

22. Use of the chimeric antigen receptor according to any one of claims 1 to 9, the polynucleotide according to claim 10, or the expression vector according to claim 11 in the detection of GPRC5D or in the diagnosis of a GPRC5D-associated disease.

23. A method for detecting GPRC5D, the method comprising:
detecting GPRC5D in a sample to be tested using at least one of the chimeric antigen receptor according to any one of claims 1 to 9, the polynucleotide according to claim 10, or the expression vector according to claim 11.

24. A method for diagnosing whether a subject is suffering from a GPRC5D-associated disease, the method comprising:
detecting GPRC5D in a sample collected from the subject using at least one of the chimeric antigen receptor according to any one of claims 1 to 9, the polynucleotide according to claim 10, or the expression vector as according to claim 11.
